(19) Europäisches Patentamt
European Patent Office
Office européen des brevets

(11) EP 3 133 390 A1

(12) EUROPEAN PATENT APPLICATION
published in accordance with Art. 153(4) EPC

(43) Date of publication:
**22.02.2017 Bulletin 2017/08**

(21) Application number: **15780046.7**

(22) Date of filing: **16.03.2015**

(51) Int Cl.:
***G01N 27/22*** *(2006.01)* ***G01N 27/02*** *(2006.01)*
***G01N 27/06*** *(2006.01)* ***G01N 33/49*** *(2006.01)*
***G01N 33/86*** *(2006.01)*

(86) International application number:
**PCT/JP2015/057647**

(87) International publication number:
**WO 2015/159623 (22.10.2015 Gazette 2015/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **17.04.2014 JP 2014085859**

(71) Applicant: **Sony Corporation**
**Tokyo 108-0075 (JP)**

(72) Inventors:
- **BRUN Marcaurele**
  **Tokyo 108-0075 (JP)**
- **MACHIDA Kenzo**
  **Tokyo 108-0075 (JP)**
- **HAYASHI Yoshihito**
  **Tokyo 108-0075 (JP)**
- **UMETSU Tomoyuki**
  **Tokyo 108-0075 (JP)**
- **KAWAGUCHI, Kaori**
  **Tokyo 108-0075 (JP)**

(74) Representative: **D Young & Co LLP**
**120 Holborn**
**London EC1N 2DY (GB)**

# (54) BLOOD STATE ANALYSIS DEVICE, BLOOD STATE ANALYSIS SYSTEM, BLOOD STATE ANALYSIS METHOD, AND PROGRAM

(57) Provided is a blood condition analysis device, a blood condition analysis system, a blood condition analysis method, and a program, according to which the coagulation system or fibrinolysis system of blood can be accurately evaluated from electrical characteristics.

In a blood condition analysis device, an analysis unit is provided for evaluating, with respect to two or more blood samples adjusted from one blood specimen and having different drug types or concentrations, the influence of the drug or a factor in the blood on the coagulation system or fibrinolysis system of the blood on the basis of data of time-dependent changes in electrical characteristics measured at a specific frequency or frequency band.

*FIG. 5*

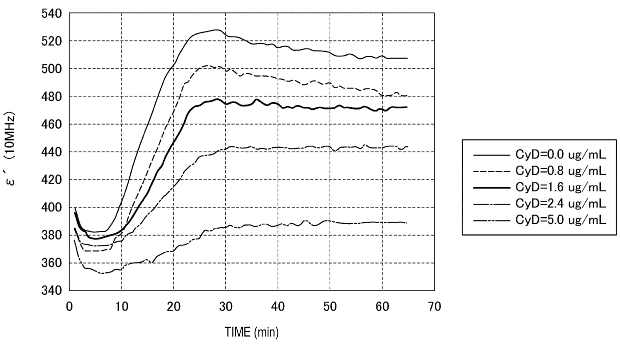


EP 3 133 390 A1

Printed by Jouve, 75001 PARIS (FR)

## Description

TECHNICAL FIELD

The present technique relates to a blood condition analysis device, a blood condition analysis system, a blood condition analysis method, and a program. More specifically, it relates to a technique for evaluating the coagulation characteristics of blood from time-dependent changes in electrical characteristics.

BACKGROUND ART

Conventionally, for the evaluation of coagulation system or fibrinolysis system, the prothrombin time (PT) and the activated partial thromboplastin time (APTT) have been used. According to these techniques, a coagulating agent is added to blood plasma prepared by centrifugation, and the time until the blood plasma starts coagulating is measured. Meanwhile, as an evaluation method using whole blood, thromboelastometry that dynamically measures changes in viscoelasticity in the process of blood coagulation is known (e.g., see Patent Documents 1 and 2).

In addition, the present inventors have proposed a technique for obtaining the information about blood coagulation from the permittivity of blood (see, e.g., Patent Document 3) . In the blood coagulation system analysis device described in Patent Document 3, the timing of the appearance of certain viscoelastic characteristics is estimated from a parameter that indicates an increase in permittivity at a frequency to be noted within a predetermined period from the removal of the anti-coagulation effect on blood imparted by the addition of citrate or the like, thereby evaluating the risk of thrombosis.

CITATION LIST

PATENT DOCUMENT

Patent Document 1: JP 2010-513905 A
Patent Document 2: JP 2010-518371 A
Patent Document 3: WO 2010/079845 A

SUMMARY OF THE INVENTION

PROBLEMS TO BE SOLVED BY THE INVENTION

Thromboelastometry is a technique in which the process of all the constituent components of blood, such as bloodplasma and platelets, interacting with each other and coagulating is observed, thereby comprehensively evaluating the blood coagulation ability. Such a technique has been commercialized by European and American companies as comprehensive coagulation tests in the acute stage, such as TEG (registered trademark) and ROTEM (registered trademark). However, these products have problems, for example, in that (1) the measurement has not been automated, and the test results depend on the measurer's manipulation, (2) the measurement is susceptible to vibration, (3) the quality control procedure is complicated, and the reagent therefor is expensive, and (4) the interpretation of output signals (thromboelastogram) requires skill. Then, these reasons are considered to be the primary cause that hinders sufficient spread.

Meanwhile, according to the blood coagulation system analysis device described in Patent Document 3, with the automation of measurement and analysis, the high measurement accuracy, and the like, the weak points of thromboelastometry described above can be overcome. In addition, changes in the condition of blood immediately after the start of measurement can also be observed. Further, this blood coagulation system analysis device makes it possible to evaluate other factors indicating reactions in the early stage of coagulation and the condition of blood, which cannot be evaluated by thromboelastometry. Therefore, currently, there is a demand for the development of a device for clinical tests, which allows for the evaluation of the coagulation system or fibrinolysis system of blood utilizing electrical characteristics, such as permittivity.

Thus, a main object of the present disclosure is to provide a blood condition analysis device, a blood condition analysis system, a blood condition analysis method, and a program, according to which the coagulation system or fibrinolysis system of blood can be accurately evaluated from electrical characteristics.

SOLUTIONS TO PROBLEMS

A blood condition analysis device according to the present disclosure includes at least an analysis unit for evaluating, with respect to two or more blood samples adjusted from one blood specimen and having different drug types or concentrations, the influence of the drug or a factor in the blood on the coagulation system or fibrinolysis system of the blood utilizing data of time-dependent changes in electrical characteristics measured at a specific frequency or frequency band.

In the blood condition analysis device, as the drug, an activator or suppressor of the coagulation system or fibrinolysis system of the blood can be used.

The analysis unit can calculate, from data of time-dependent changes in the electrical characteristics of each blood sample, at least one kind of value selected from the group consisting of the coagulation time, the clot formation time, the maximum clot firmness, the maximum lysis, the coagulation amplitude, the coagulation rate, the clot firmness after a predetermined period of time from the coagulation time, the clot firmness after a predetermined period of time from the maximum firmness, and the clot linear velocity after the maximum firmness, andperformthe evaluation on the basis of the calculated value.

In that case, the analysis unit may correct the calculated value with the hematocrit of the blood sample.

In addition, the analysis unit can also evaluate one or both of the influence of platelet factors and the influence of fibrinogen on the coagulation system of the blood.

In that case, as the drug, a platelet function suppressor, a fibrinogen function suppressor, or a fibrin polymerization suppressor can be used.

Further, for example, the analysis unit can evaluate the influence of the drug on the basis of the difference in data of time-dependent changes in electrical characteristics or the difference in values calculated from the data of time-dependent changes between a blood sample not containing the drug and a blood sample containing the drug at an arbitrary concentration.

In addition, for example, the analysis unit can also estimate the minimum drug dose to eliminate the influence of a target factor contained in the blood sample.

On the other hand, the analysis unit can also evaluate the influence of plasmin or plasminogen on the fibrinolysis system of the blood.

In that case, as the drug, an activator or inhibitor of plasminogen or plasmin can be used.

Further, for example, the analysis unit can evaluate the influence of plasmin or plasminogen by comparing data of time-dependent changes in electrical characteristics between a blood sample not containing the drug and a blood sample containing the drug at an arbitrary concentration.

Further, the blood condition analysis device of the present disclosure uses, as the drug, heparin, and the analysis unit may evaluate the suppressing effect of the heparin on blood coagulation.

A blood condition analysis system according to the present disclosure includes: an electrical characteristic measurement device including a measurement unit for measuring the electrical characteristics of two or more blood samples adjusted from one blood specimen and having different drug types or concentrations over time at a specific frequency or frequency band; and a blood condition analysis device including an analysis unit for evaluating the influence of the drug or a factor in the blood on the coagulation system or fibrinolysis system of the blood utilizing data of time-dependent changes in the electrical characteristics measured by the electrical characteristic measurement device.

The blood condition analysis system of the present disclosure may further include a server including an information storage unit for storing the measurement data from the electrical characteristic measurement device and/or the analysis results from the blood condition analysis device, and the server may be connected to the electrical characteristic measurement device and/or the blood condition analysis device through a network.

A blood condition analysis method according to the present disclosure includes: a measurement step of measuring the electrical characteristics of two or more blood samples adjusted from one blood specimen and having different drug types or concentrations over time at a specific frequency or frequency band; and an analysis step of evaluating the influence of the drug or a factor in the blood on the coagulation system or fibrinolysis system of the blood utilizing data of time-dependent changes in the electrical characteristics measured in the measurement step.

A program according to the present disclosure is for causing a computer to implement an analysis function of evaluating, with respect to two or more blood samples adjusted from one blood specimen and having different drug types or concentrations, the influence of the drug or a factor in the blood on the coagulation system or fibrinolysis system of the blood utilizing data of time-dependent changes in electrical characteristics measured at a specific frequency or frequency band.

EFFECTS OF THE INVENTION

According to the present disclosure, the coagulation system or fibrinolysis system of blood can be accurately evaluated from data of time-dependent changes in electrical characteristics. Incidentally, the effects described herein are not necessarily limited, and may be any of the effects described in the present disclosure.

BRIEF DESCRIPTION OF DRAWINGS

Fig. 1 is a diagram showing the schematic configuration of a blood condition analysis system of a first embodiment of the present disclosure.

Fig. 2 is graphs as a substitute for a drawing, showing time-dependent changes in the complex permittivity accompanying blood coagulation, wherein A shows the real part (ε'), while B shows the imaginary part (ε").

Fig. 3 is a graph as a substitute for a drawing, comparing, with respect to the clot strength of blood, a value determined by viscoelasticity measurement and a value determined from the permittivity.

Fig. 4 is a flow chart diagram showing an operation example of the blood condition analysis device shown in Fig. 1.

Fig. 5 is a graph as a substitute for a drawing, showing time-dependent changes in the real part (ε') of the complex permittivity of blood samples having different CyD concentrations at 10 MHz.

Fig. 6 is a graph as a substitute for a drawing, showing, with respect to the real part (ε') of the complex permittivity at 10 MHz shown in Fig. 5, the difference between the value of a blood sample having a CyD concentration of 0 μg/mL and the values of other blood samples.

Fig. 7 is a graph as a substitute for a drawing, showing the relation between the coagulation amplitude determined from the real part (ε') of the complex permittivity and the CyD concentration.

Fig. 8 is a graph as a substitute for a drawing, showing the relation between the coagulation rate determined from the real part (ε') of the complex permittivity and the CyD concentration.

Fig. 9 is a graph as a substitute for a drawing, showing time-dependent changes in the real part (ε') of the complex permittivity at 10 MHz of blood samples having different fibrinolysis system promotor (tPA) and fibrinolysis system inhibitor (aprotinin) concentrations.

Fig. 10 is a graph as a substitute for a drawing, showing the values of evaluation parameters calculated from the values of ε' at 10 MHz shown in Fig. 9.

Fig. 11 is a graph as a substitute for a drawing, showing time-dependent changes in the real part (ε') of the complex permittivity of blood samples having different heparin concentrations at 10 MHz.

Fig. 12 is a graph as a substitute for a drawing, showing the relation between the clotting time determined from the values of ε' at 10 MHz shown in Fig. 11 and the heparin concentration.

Fig. 13 is a graph as a substitute for a drawing, showing the coagulation curves of blood samples having different heparin concentrations.

Fig. 14 is a graph as a substitute for a drawing, showing the relation between the heparin concentration and the difference in clotting time.

In Fig. 15, A is a diagram showing the relation between the fibrinogen inhibitor (Pefabloc) concentration and the coagulation amplitude, while B is a graph as a substitute for a drawing, showing the relation with the coagulation time.

Fig. 16 is a flow chart diagram showing another operation example of the blood condition analysis device shown in Fig. 1.

Fig. 17 is a graph as a substitute for a drawing, showing time-dependent changes in the real part (ε') of the complex permittivity of blood samples having different PLTs and HCTs at 1 MHz.

Fig. 18 is a graph as a substitute for a drawing, showing time-dependent changes in the real part (ε') of the complex permittivity of blood samples having different PLTs and HCTs at 10 MHz.

Fig. 19 shows coagulation curves of the blood samples shown in Fig. 18 with the addition of excess CyD.

Fig. 20 is a graph as a substitute for a drawing, showing the difference between the values shown in Fig. 18 and the values shown in Fig. 19.

Fig. 21 is a graph as a substitute for a drawing, showing the relation between the coagulation rate estimated from the real part (ε') of the complex permittivity and the platelet concentration.

Fig. 22 is a graph as a substitute for a drawing, showing the relation between the coagulation amplitude estimated from the real part (ε') of the complex permittivity and the platelet concentration.

Fig. 23 is a graph as a substitute for a drawing, showing the relation between the coagulation amplitude estimated from the real part (ε') of the complex permittivity and corrected with HCT and the effective concentration of platelets.

Fig. 24 is a graph as a substitute for a drawing, showing, with respect to several specimens having different Pefabloc concentrations, the results of examining the relation between the CyD concentration and the coagulation amplitude.

Fig. 25 is a graph as a substitute for a drawing, showing, with respect to several specimens having different CyD concentrations, the results of examining the relation between the Pefabloc concentration and the coagulation amplitude.

Fig. 26 is a graph as a substitute for a drawing, showing an example of time-dependent changes in the real part of the complex permittivity accompanying blood coagulation.

Fig. 27 is a graph as a substitute for a drawing, showing the measurement results fromblood samples without the addition of Pefabloc.

Fig. 28 is a graph as a substitute for a drawing, showing the relation between the amplitude CF of a blood sample having the function of platelets completely suppressed with CyD and the effective concentration of fibrinogen FIB.

Fig. 29 is a graph as a substitute for a drawing, showing the relation between the amplitude CF - a x the effective concentration of fibrinogen FIB and the effective concentration of platelets PLT.

Fig. 30 is a graph as a substitute for a drawing, showing the relation between the amplitude CF - a x the effective concentration of fibrinogen FIB and the effective concentration of platelets PLT.

Fig. 31 is a graph as a substitute for a drawing, prepared by plotting the CyD concentration on the horizontal axis and, on the vertical axis, a value obtained by subtracting the amplitude of a blood sample having the function of platelets completely suppressed with CyD (CF-CyDY) from the amplitude at each CyD concentration (CF-CyDX).

Fig. 32 is a graph as a substitute for a drawing, showing the relation between the inclination at each Pefabloc concentration and the effective concentration of fibrinogen measured by a fibrinogen measurement device (trade name: "DRIHemato" (registered trademark), manufactured by A&T).

Fig. 33 is a graph as a substitute for a drawing, showing, in the case of using the effective concentration of fibrinogen measured by a fibrinogen measurement device (trade name: "DRIHemato" (registered trademark), manufactured by A&T), the correlation between the amplitude CF calculatedusing the above Mathematical Formula 9 from measurement results different from the results used in Examination of Model (1) and the actual measured amplitude CF.

Fig. 34 is a graph as a substitute for a drawing, showing, in the case of using the effective concentration of fibrinogen calculated from Fig. 28, the correlation between the amplitude CF calculated using the above Mathematical Formula 9 from measurement results different from the results used in Examination of Model (1) and the actual measured amplitude CF.

Fig. 35 is a graph as a substitute for a drawing, showing the relation between the initial value of the complex permittivity and the hematocrit.

Fig. 36 is a graph as a substitute for a drawing, showing the comparison data corresponding to Fig. 33 corrected using the hematocrit measured by a multiparameter automated hematology analyzer.

Fig. 37 is a graph as a substitute for a drawing, showing the comparison data corresponding to Fig. 34 corrected using the hematocrit measured by a multiparameter automated hematology analyzer.

Fig. 38 is a graph as a substitute for a drawing, showing the comparison data corresponding to Fig. 33 corrected using the hematocrit calculated from the initial value of the complex permittivity.

Fig. 39 is a graph as a substitute for a drawing, showing the comparison data corresponding to Fig. 34 corrected using the hematocrit calculated from the initial value of the complex permittivity.

MODE FOR CARRYING OUT THE INVENTION

Hereinafter, modes for carrying out the present disclosure will be described in detail with reference to the attached drawings. Incidentally, the present disclosure is not limited to the embodiments shown below. In addition, the present disclosure will be described in the following order.

1. First Embodiment

(Example of a blood condition analysis system for evaluating the coagulation system/fibrinolysis system of blood from electrical characteristics)

2. Variation of First Embodiment

(Example of a blood condition analysis system, including correction with HCT)

(1. First Embodiment)

First, a blood condition analysis system according to the first embodiment of the present disclosure will be described. Fig. 1 is a diagram showing the schematic configuration of the blood condition analysis system of this embodiment. As shown in Fig. 1, the blood condition analysis system 1 of this embodiment is provided with an electrical characteristic measurement device 10 and a blood condition analysis device 11. In addition, as necessary, the blood condition evaluation system of this embodiment may also have connected thereto a server 12, a display device 13, and the like.

[Electrical Characteristic Measurement Device 10]

The electrical characteristic measurement device 10 includes a measurement unit that applies a voltage between a pair of electrodes provided in a sample container filled with a blood sample to be evaluated, and measures the electrical characteristics of the blood sample over time at a specific frequency or frequency band. Examples of electrical characteristics measured by the electrical characteristic measurement device 10 include the impedance, conductance, admittance, capacitance, permittivity, conductivity, and phase angle, as well as amounts obtained by converting them into the amounts of electricity. Incidentally, the blood condition analysis system 1 of this embodiment can perform evaluation from at least one kind of these electrical characteristics, but may also utilize two or more kinds of electrical characteristics.
The configuration of the electrical characteristic measurement device 10 is not particularly limited, and may be suitably set according to the electrical characteristics to be measured. For example, in the case where an alternating voltage is applied between a pair of electrodes to measure the impedance or complex permittivity of blood, it is also possible to use an impedance analyzer or a network analyzer. Incidentally, although the electrical characteristic measurement device 10 may measure the characteristics only at the frequency or frequency band utilized in the blood condition analysis device 11 described below, it is also possible that the frequency is changed to measure the electrical characteristics at a wider band, and then the frequency or frequency band utilized for evaluation is extracted from the obtained spectra.

[Blood Condition Analysis Device 11]

The blood condition analysis device 11 is connected to the electrical characteristic measurement device 10 directly or through a network 14, and includes an analysis unit that analyzes the influence of a drug or a factor in blood on the coagulation characteristics or fibrinolysis characteristics of the blood. The blood condition analysis device 11 receives the input of data of time-dependent changes in electrical characteristics measured by the electrical characteristic measurement device 10, and the analysis unit performs evaluation utilizing the data of time-dependent changes in electrical characteristics.

[Server 12]

The server 12 is connected, for example, through the network 14, to the electrical characteristic measurement device 10, the blood condition analysis device 11, the display device 13, and the like, and is provided with an information storage unit and the like. Then, the server 12 controls the various data uploaded from the electrical characteristic measurement device 10 and the blood condition analysis device 11, and outputs the same to the display device 13 or the blood condition analysis device 11 as required.

[Display Device 13]

The display device 13 displays data of the electrical characteristics of a blood sample measured by the electrical characteristic measurement device 10, the evaluation results from the blood condition analysis device 11, and the like. Incidentally, the display device 13 may be provided with an information input unit for the user to select and input the data to be displayed. In this case, the information input by the user is sent to the server 12 or the blood condition analysis device 11 through the network 14.

[Operation]

Next, the operation of the blood condition analysis system of this embodiment, that is, a method for evaluating the coagulation system or fibrinolysis system of a blood sample using the blood condition analysis system, will be described. Fig. 2 is a diagram showing time-dependent changes in the complex permittivity accompanying blood coagulation, wherein A shows the real part ($\varepsilon'$), while B shows the imaginary part ($\varepsilon''$). In addition, Fig. 3 is a diagram that compares, with respect to the clot strength of blood, a value determined by viscoelasticity measurement and a value determined from the permittivity.
For example, as shown in Fig. 2A and Fig. 2B, from data of time-dependent changes in the complex permittivity of blood, the coagulation start time a and the coagulation end time b can be estimated. In addition, the difference in amplitude between the real part and imaginary part of the complex permittivity is equivalent to the coagulation amplitude, and the inclination of the line connecting between the coagulation start time a and the coagulation end time b is equivalent to the coagulation rate. Then, as shown in Fig. 3, the value of the clot strength of blood estimated from the complex permittivity is correlated with the value of the clot strength determined by viscoelasticity measurement, such as thromboelastometry.

Thus, according to the blood condition analysis system of this embodiment, with respect to two or more blood samples adjusted from one blood specimen and having different drug types or concentrations, the influence of the drug or a factor in the blood on the coagulation system or fibrinolysis system of the blood is evaluated. At this time, for the evaluation, data of time-dependent changes in electrical characteristics measured at a specific frequency or frequency band is utilized. Fig. 4 is a flow chart diagram showing an operation example of the blood condition analysis system 1 shown in Fig. 1.

Specifically, as shown in Fig. 4, first, by the electrical characteristic measurement device 10, the electrical characteristics of two or more blood samples having different drug concentrations are measured over time at a specific frequency or frequency band (measurement step S1). Subsequently, utilizing data of time-dependent changes in the electrical characteristics measured by the electrical characteristic measurement device 10, the influence of the drug or a factor in the blood on the coagulation system or fibrinolysis system of the blood is evaluated by the blood condition analysis device 11 (analysis step S2).

(Measurement Step S1)

In the measurement step S1, the electrical characteristics of two or more blood samples adjusted from one blood specimen and having different drug types or concentrations are measured over time at a specific frequency or frequency band. At this time, the conditions for the electrical characteristic measurement are not particularly limited, and may be suitably set according to the kind of electrical characteristics and the like within a range where the blood to be evaluated is not deteriorated.

In addition, although the measurement may be performed only at the frequency or frequency band utilized in the analysis step described below, it is also possible that the electrical characteristics are measured at a wide band including all the frequencies and frequency bands utilized. In that case, the frequency or frequency band utilized for evaluation may be extracted from the obtained spectra in the blood condition analysis device 11. Specifically, as the frequency band at which the measurement is performed, a range of 100 Hz to 100 MHz, where the measurement is less affected by protein, is preferable, and a range of 1 kHz to 10 MHz is more preferable.

Meanwhile, as a drug to be added to the blood sample, an activator or suppressor of the coagulation system or fibrinolysis system of blood may be used. Specific examples thereof include platelet function suppressors such as cytochalasin D (hereinafter abbreviated to CyD), fibrinogen function suppressors such as H-Gly-Pro-Arg-Pro-OH x AcOH (PefablocFG), a fibrin polymerization suppressor, a plasminogen activator, plasmin suppressors such as aprotinin and tranexamic acid, and coagulation suppressors such as heparin.

(Analysis Step S2)

In the analysis step S2, utilizing data of time-dependent changes in the electrical characteristics measured by the electrical characteristic measurement device 10, the influence of the drug or a factor in the blood on the coagulation system or fibrinolysis system of the blood is evaluated by the blood condition analysis device 11. For example, from data of time-dependent changes in the electrical characteristic of each blood sample, parameters conventionally used for the evaluation of the coagulation system or fibrinolysis system of blood are calculated. Here, examples of parameters for the evaluation of the coagulation system or fibrinolysis system of blood include clotting time, clot formation time, maximum clot firmness, maximum lysis, coagulation amplitude, coagulation rate, clot firmness after a predetermined period of time from the coagulation time, clot firmness after a predetermined period of time from the maximum firmness, and clot linear velocity after the maximum firmness.

In addition, for example, in the case where the coagulation system is evaluated, examples of coagulation factors to be evaluated include platelet factors and fibrinogen. In that case, as a drug to be added to the blood sample, a platelet function suppressor, a fibrinogen function suppressor, a fibrin polymerization suppressor, or the like is usable. Then, the influence thereof can be evaluated, for example, on the basis of the difference in coagulation amplitude or coagulation rate determined from data of time-dependent changes in the electrical characteristics of a blood sample not containing the drug and a blood sample containing the drug at an arbitrary concentration.

Fig. 5 is a diagram showing time-dependent changes in the real part (ε') of the complex permittivity of blood samples having different CyD concentrations at 10 MHz. In addition, Fig. 6 is a diagram showing, with respect to the real part (ε') of the complex permittivity at 10 MHz shown in Fig. 5, the difference between the value of a blood sample having a CyD concentration of 0 μg/ml and the values of other blood samples. As shown in Fig. 5, it can be seen that the value of ε' at 10 MHz and its time-dependent change characteristics vary depending on the CyD concentration. In addition, as shown in Fig. 6, it can be seen that the higher the CyD concentration, the greater the influence.

Fig. 7 is a diagram showing the relation between the coagulation amplitude determined from the real part (ε') of the complex permittivity and the CyD concentration. Fig. 8 is a diagram showing the relation between the coagulation rate determined from the real part (ε') of the complex permittivity (= (amplitude B - amplitude A) / (time B - time A)) and the CyD concentration. As shown in Fig. 7 and Fig. 8, it can be seen that the values of the coagulation amplitude and coagulation rate determined from the real part (ε') of the complex permittivity are correlated with the CyD concentration of the blood sample.

Then, from the evaluation results, the minimum drug dose to completely eliminate the influence of the target factor contained in the blood can be estimated. For example, in the case of CyD, the coagulation rate becomes constant when a certain concentration is reached or exceeded. Thus, it is estimated that 3.3 μg/ml, which agrees with the coagulation rate of a blood sample containing excess CyD indicated with a broken line in Fig. 8, is the minimum drug dose to completely suppress the action of platelet factors. In addition, in the case of the blood sample shown in Fig. 8, the number of platelets is 136500 per 1 μl, and thus the amount of CyD for inhibiting the platelet factors is 25 ng per platelet.

In addition, for example, in the case where the fibrinolysis system is evaluated, examples of coagulation factors to be evaluated include plasminogen and plasmin. In that case, as a drug to be added to the blood sample, an activator or inhibitor of plasminogen or plasmin is usable. Fig. 9 is a diagram showing time-dependent changes in the real part (ε') of the complex permittivity at 10 MHz of blood samples having different fibrinolysis system promotor (tPA) and fibrinolysis system inhibitor (aprotinin) concentrations.

Fig. 9 shows values measured using fibrinolysis-system blood samples artificially prepared by adding tPA, which is a fibrinolysis system promotor, to blood. The blood sample of tPA = 8 and aprotinin = 0 exhibits similar characteristics to the blood of a patient with the fibrinolysis system promoted. As shown in Fig. 9, it can be seen that the value of ε' at 10 MHz and its time-dependent change characteristics vary depending on the aprotinin concentration. Incidentally, Fig. 9 also shows the measurement results of a control blood sample (tPA = 0, aprotinin = 0) without the addition of the drug.

For the evaluation of the fibrinolysis-system blood, for example, a value obtained by dividing the amplitude after 30 minutes from the time B shown in Fig. 2 by the amplitude at the time B (LI30: the source of clot firmness after a predetermined period of time from the maximum firmness accompanying fibrinolysis) can be used as an evaluation parameter. Fig. 10 is a diagram showing the values of evaluation parameters calculated from the values of ε' at 10 MHz shown in Fig. 9. As shown in Fig. 10, it can be seen that as a result of adding aprotinin, which is a fibrinolysis system inhibitor, to blood, the value of LI30, which is an evaluation parameter, turns back to the value of the control blood sample without the addition of the drug.

Incidentally, the evaluation of the fibrinolysis system is not limited to the method described above, and may also be performed at a frequency other than 10 MHz, such as 100 kHz. In addition, the evaluation may also use a parameter associated with the fibrinolysis system, and values other than LI30 are also usable.

The blood condition analysis method of this embodiment is not limited to the evaluation criteria and evaluation methods described above. For example, it is also possible that heparin is used as the drug, and the suppressing effect of heparin on blood coagulation is evaluated. Fig. 11 is a diagram showing time-dependent changes in the real part (ε') of the complex permittivity of blood samples having different heparin concentrations at 10 MHz, and Fig. 12 is a diagram showing the relation between the clotting time determined from the values of ε' at 10 MHz shown in Fig. 11 and the heparin concentration. In addition, Fig. 13 is a diagram showing the coagulation curves of blood samples having different heparin concentrations, and Fig. 14 is a diagram showing the relation between the heparin concentration and the difference in clotting time.

As shown in Fig. 11, it can be seen that the value of ε' at 10 MHz and its time-dependent change characteristics vary depending on the heparin concentration. In addition, as shown in Fig. 12, it can be seen that the clotting time increases with an increase in the heparin concentration, and the higher the heparin concentration, the greater the influence. Thus, the influence of heparin can be evaluated from the clotting time shown in Fig. 13, for example. Specifically, as shown in Fig. 14, the difference in clotting time between a blood sample with the addition of heparin and a blood sample without the addition of heparin is determined, whereby the amount of heparin in a blood sample can be estimated.

Further, by using a fibrinogen inhibitor, such as Pefabloc, as the drug, the influence of fibrinogen can also be evaluated. Fig. 15A is a diagram showing the relation between the fibrinogen inhibitor (Pefabloc) concentration and the coagulation amplitude, while Fig. 15B is a diagram showing the relation with the coagulation time. As shown in Fig. 15A and Fig. 15B, the coagulation amplitude and the clotting time change with an increase in the fibrinogen inhibitor (Pefabloc) concentration of the blood sample. Thus, utilizing this characteristic, the influence of fibrinogen can be evaluated, or the amount of fibrinogen can be estimated.

In the analysis step S2, a computer program for implementing each of the functions described above can be created and installed on a personal computer or the like. Such a computer program may be stored, for example, in a recording media such as a magnetic disk, an optical disc, a magneto-optical disc, or a flash memory, or may also be distributed through a network.

According to the blood condition analysis system of this embodiment, evaluation is performed using data of time-dependent changes in electrical characteristics, whereby the influence of a specific factor, which cannot be observed with a conventional evaluation method, can be evaluated. This makes it possible to obtain evaluation results that are more reliable than before. In addition, according to the blood condition analysis system of this embodiment, the coagulation system or fibrinolysis system of blood can be accurately evaluated in a simple manner by short-time measurement.

Incidentally, although the measurement device and the analyzing device are separately provided in the blood condition analysis system of this embodiment, they may also be integrated. In addition, the analysis device may also perform evaluation on the basis of the previously measured data.

(2. Variation of First Embodiment)

According to the blood condition analysis system of the present disclosure, although the parameters estimated from data of time-dependent changes in electrical characteristics may be directly used for evaluation, it is also possible that data of the estimated values is corrected using the hematocrit (HCT) of the blood. Fig. 16 is a flow chart diagram showing another operation example of the blood condition analysis device of this variation. Specifically, as shown in Fig. 16, according to the blood condition analysis system of this variation, data correction is performed in the analysis step S2.

Fig. 17 is a diagram showing time-dependent changes in the real part (ε') of the complex permittivity of blood samples having different PLTs and HCTs at 1 MHz. Fig. 18 is a diagram showing time-dependent changes in the real part (ε') of the complex permittivity of blood samples having different PLTs and HCTs at 10 MHz. Fig. 19 shows coagulation curves of the blood samples shown in Fig. 18 with the addition of excess CyD. From this diagram, the process of coagulation in the state where the influence of platelets has been completely eliminated can be observed. Fig. 20 is a diagram showing the difference between the values shown in Fig. 18 and the values shown in Fig. 19. From this diagram, the influence of platelets and the influence of the interaction between fibrinogen and platelets on coagulation can be observed.

As shown in Fig. 17 and Fig. 18, the value of ε' at 10 MHz and its time-dependent change characteristics vary depending on HCT. Then, from the results shown in Fig. 17 to Fig. 20, it can be seen that in blood samples having the equivalent HCT level, there is a correlation between the number of platelets and the coagulation amplitude or the coagulation rate. However, when the HCT changes, even when the number of platelets is on the equivalent level, the coagulation amplitude increases. In addition, when the HCT is low, and the number of platelets is large, the coagulation amplitude hardly changes. From this, the correction with HCT appears to be effective.

Fig. 21 is a diagram showing the relation between the coagulation rate estimated from the real part (ε') of the complex permittivity and the platelet concentration. Fig. 22 is a diagram showing the relation between the coagulation amplitude estimated from the real part (ε') of the complex permittivity and the platelet concentration. In Fig. 21 and Fig. 22, the data indicated with circles (O) are data without the addition of CyD extracted from the data of Fig. 17, while the data indicated with X are data with the addition of excess CyD extracted from the data of Fig. 18. Then, the data indicated with squares (□) in Fig. 21 and Fig. 22 show the difference between them, that is, the influence of platelets and the influence of the interaction between fibrinogen and platelets on coagulation.

Incidentally, in this technique, "effective concentration" refers to the concentration of molecules, cells, and the like that are actually functioning in the predetermined reaction. For example, although the concentration of platelets simply means the number of platelets contained in a certain blood specimen, the effective concentration of platelets in a blood coagulation reaction means the number of platelets that actually function in the blood coagulation reaction. That is, in the case where a drug that suppresses the function of platelets, for example, is added, the effective concentration of platelets decreases.

As shown in Fig. 21 and Fig. 22, the data indicated with circles and squares is dependent on the number of platelets. Incidentally, the data surrounded by broken lines in Fig. 21 and Fig. 22 is corresponding to the blood samples having a small number of platelets and blood samples having a large number of platelets shown in Fig. 17 to Fig. 19. These blood samples are different in HCT from other blood samples, and, as a result, the correlations of the coagulation rate and amplitude with the number of platelets are also deviated. Therefore, the data of these blood samples needs to be corrected.

Fig. 23 shows the values in Fig. 21 corrected with HCT (normalized with the fourth power of HCT). As shown in Fig. 23, as a result of correcting the data of Fig. 21 with HCT, the relation with the number of platelets has become a straight line. In addition, in the case where the difference in coagulation between with and without CyD is determined from one specimen, the HCT and the number of platelets can be estimated by the dielectric measurement of the two coagulation curves.

As a result of correction with HCT in this manner, the accuracy of the evaluation results from the analysis device can be further improved. Incidentally, the configurations and effects of this variation other than those described above are similar to those in the first embodiment described above.

In addition, the present disclosure may also be configured as follows.

(1) A blood condition analysis device, including at least an analysis unit for evaluating, with respect to two or more blood samples adjusted from one blood specimen and having different drug types or concentrations, the influence of the drug or a factor in the blood on the coagulation system or fibrinolysis system of the blood utilizing data of time-dependent changes in electrical characteristics measured at a specific frequency or frequency band.

(2) The blood condition analysis device according to (1), wherein the drug is an activator or suppressor of the coagulation system or fibrinolysis system of the blood.

(3) The blood condition analysis device according to (1) or (2), wherein the analysis unit calculates, from data of time-dependent changes in the electrical characteristics of each blood sample, at least one kind of value selected from the group consisting of the coagulation time, the clot formation time, the maximum clot firmness, the maximum lysis, the coagulation amplitude, the coagulation rate, the clot firmness after a predetermined period of time from the coagulation time, the clot firmness after a predetermined period of time from the maximum firmness, and the clot linear velocity after the maximum firmness, and performs the evaluation on the basis of the calculated value.

(4) The blood condition analysis device according to (3), wherein the analysis unit corrects the calculated value with the hematocrit of the blood sample.

(5) The blood condition analysis device according to any of (1) to (4), wherein the analysis unit evaluates one or both of the influence of platelet factors and the influence of fibrinogen on the coagulation system of the blood.

(6) The blood condition analysis device according to (5), wherein the drug is a platelet function suppressor, a fibrinogen function suppressor, or a fibrin polymerization suppressor.

(7) The blood condition analysis device according to (5) or (6), wherein the analysis unit evaluates the influence of the drug on the basis of the difference in data of time-dependent changes in electrical characteristics or the difference in values calculated from the data of time-dependent changes between a blood sample not containing the drug and a blood sample containing the drug at an arbitrary concentration.

(8) The blood condition analysis device according to any of (5) to (7), wherein the analysis unit estimates the minimum drug dose to eliminate the influence of a target factor contained in the blood sample.

(9) The blood condition analysis device according to any of (1) to (4), wherein the analysis unit evaluates the influence of plasmin or plasminogen on the fibrinolysis system of the blood.

(10) The blood condition analysis device according to (9), wherein the drug is an activator or inhibitor of plasminogen or plasmin.

(11) The blood condition analysis device according to (9) or (10), wherein the analysis unit evaluates the influence of plasmin or plasminogen by comparing data of time-dependent changes in electrical characteristics between a blood sample not containing the drug and a blood sample containing the drug at an arbitrary concentration.

(12) The blood condition analysis device according to any of (1) to (4), wherein the drug is heparin, and the analysis unit evaluates the suppressing effect of the heparin on blood coagulation.

(13) A blood condition analysis system, including: an electrical characteristic measurement device including a measurement unit for measuring the electrical characteristics of two or more blood samples adjusted from one blood specimen and having different drug types or concentrations over time at a specific frequency or frequency band; and a blood condition analysis device including an analysis unit for evaluating the influence of the drug or a factor in the blood on the coagulation system or fibrinolysis system of the blood utilizing data of time-dependent changes in the electrical characteristics measured by the electrical characteristic measurement device.

(14) The blood condition analysis system according to (13), further including a server including an information storage unit for storing the measurement data from the electrical characteristic measurement device and/or the analysis results from the blood condition analysis device, the server being connected to the electrical characteristic measurement device and/or the blood condition analysis device through a network.

(15) A blood condition analysis method, including: a measurement step of measuring the electrical characteristics of two or more blood samples adjusted from one blood specimen and having different drug types or concentrations over time at a specific frequency or frequency band; and an analysis step of evaluating the influence of the drug or a factor in the blood on the coagulation system or fibrinolysis system of the blood utilizing data of time-dependent changes in the electrical characteristics measured in the measurement step.

(16) A program for causing a computer to implement an analysis function of evaluating, with respect to two or more blood samples adjusted from one blood specimen and having different drug types or concentrations, the influence of the drug or a factor in the blood on the coagulation system or fibrinolysis system of the blood utilizing data of time-dependent changes in electrical characteristics measured at a specific frequency or frequency band.

Incidentally, the effects described herein are merely examples and not restrictive, and there may also be other effects.

Example 1

Hereinafter, the effects of the present disclosure will be described in detail. In this example, CyD or Pefabloc was added at various concentrations to a blood sample collected from a healthy individual, and changes in the complex permittivity in the process of coagulation were measured. The CyD concentration, the Pefabloc concentration, and the like of each blood sample are shown in the following Table 1. In addition, Fig. 24 shows, with respect to several specimens having different Pefabloc concentrations, the results of examining the relation between the CyD concentration and the coagulation amplitude, and Fig. 25 shows, with respect to several specimens having different CyD concentrations, the results of examining the relation between the Pefabloc concentration and the coagulation amplitude.

[Table 1]

| No. | Blood (μl) | CyD (μl) | PFG (μl) | PBS (μl) | CyD (μg/ml) | PFG (μg/ml) |
|---|---|---|---|---|---|---|
| 1 | 540.00 | 0.00 | 0.00 | 48.00 | 0.00 | 0.00 |
| 2 | 540.00 | 6.00 | 0.00 | 42.00 | 0.86 | 0.00 |
| 3 | 540.00 | 18.00 | 0.00 | 30.00 | 2.57 | 0.00 |
| 4 | 540.00 | 36.00 | 0.00 | 12.00 | 5.14 | 0.00 |
| 5 | 540.00 | 0.00 | 3.00 | 45.00 | 0.00 | 510.20 |
| 6 | 540.00 | 6.00 | 3.00 | 39.00 | 0.86 | 510.20 |
| 7 | 540.00 | 18.00 | 3.00 | 27.00 | 2.57 | 510.20 |
| 8 | 540.00 | 36.00 | 3.00 | 9.00 | 5.14 | 510.20 |
| 9 | 540.00 | 0.00 | 6.00 | 42.00 | 0.00 | 1020.41 |
| 10 | 540.00 | 6.00 | 6.00 | 36.00 | 0.86 | 1020.41 |
| 11 | 540.00 | 18.00 | 6.00 | 24.00 | 2.57 | 1020.41 |
| 12 | 540.00 | 36.00 | 6.00 | 6.00 | 5.14 | 1020.41 |
| 13 | 540.00 | 0.00 | 12.00 | 36.00 | 0.00 | 2040.82 |
| 14 | 540.00 | 6.00 | 12.00 | 30.00 | 0.86 | 2040.82 |
| 15 | 540.00 | 18.00 | 12.00 | 18.00 | 2.57 | 2040.82 |
| 16 | 540.00 | 36.00 | 12.00 | 0.00 | 5.14 | 2040.82 |

As shown in the following Mathematical Formula 1, the clot firmness CF depends on the effective concentration of platelets PLT and the effective concentration of fibrinogen FIB. Incidentally, a, b, and c in the following Mathematical Formula 1 are parameters determined by measurement.

[Mathematical Formula 1]

$$CF = a \times PLT + b \times FIB + c \times (PLT \times FIB)$$

Here, by applying the analysis method shown in Fig. 8 to the data of a sample having a Pefabloc concentration of 0% shown in Fig. 24, it can be estimated that the CyD concentration at which the contribution of platelets disappears is 3.4 μg/ml (the addition of 8 μl of CyD to 180 μl of blood). As a result, the effective concentration of platelets (142000/μl) can be determined. In a similar manner, the amount of fibrinogen can be determined using the data of a sample having a CyD concentration of 0% shown in Fig. 25.

In the case where there is no contribution of platelets, the coagulation amplitude is about 105. Accordingly, the following Mathematical Formula 2 is derived from the above Mathematical Formula 1.

[Mathematical Formula 2]

$$105 = b \times FIB$$

In addition, in the case where the CyD concentration is 0, and the amount of fibrinogen is excess, the following Mathematical Formula 3 is derived from the above Mathematical Formula 1.

[Mathematical Formula 3]

$$105 = a \times PLT$$

Incidentally, in the case where neither fibrinogen nor CyD is added, the amplitude is 128. Accordingly, the above Mathematical Formula 1 is represented by the following Mathematical Formula 4, and the following Mathematical Formula 5 is derived from the following Mathematical Formula 4.

[Mathematical Formula 4]

$$128 = 105 + 105 + c \times \{(105/a) \times (105/b)\}$$

[Mathematical Formula 5]

$$c = (128 - 210)/(105 \times 105) \times a \times b$$

Then, the effective concentration of platelets PLT can be estimated from data of CyD concentration dependence. In addition, the effective concentration of fibrinogen FIB can be estimated from data of PFG concentration dependence. Meanwhile, when there is data of the addition of excess CyD, and the parameter b is known beforehand, the effective concentration of fibrinogen FIB can be estimated from the above Mathematical Formula 2. In addition, when there is data of the addition of excess Pefabloc, and the parameter a is known beforehand, the effective concentration of platelets PLT can be estimated from the above Mathematical Formula 3.

Further, in addition to data of CyD concentration dependence and excess addition, when there is data of Pefabloc concentration dependence and excess addition and also data of the addition of neither, a, b, and c can be estimated from the above Mathematical Formula 5.

In this example, the effective concentration of platelets PLT is 3. 4 μg/ml (equivalent CyD), and the effective concentration of fibrinogen FIB is 1000 μg/ml (equivalent PFG). Accordingly, a is 30.88 ml/MG (eq CyD), b is 0.105 ml/MG (eq PFG), and c is -0.024 ml/MG (eq CyD).

In addition, with respect to the sample No. 6 shown in the above Table 1, the above Mathematical Formula 1 is represented by the following Mathematical Formula 6 in the case of not adding CyD or by the following Mathematical Formula 7 in the case of adding excess CyD.

[Mathematical Formula 6]

$$109.6 = 30.88 \times PLT + 0.105 \times FIB - 0.024 \times PLT \times FIB$$

[Mathematical Formula 7]

$$101.2 = 0.105 \times FIB$$

From the above Mathematical Formula 6 and Mathematical Formula 7, the effective concentration of fibrinogen FIB is calculated to be about 9640 μg/ml (equivalent PFG), and the effective concentration of platelets PLT is calculated to be about 1.1 μg/ml (equivalent CyD).

Example 2

In Example 2, in the measurement of blood coagulation using electrical characteristics, the influences of the effective concentrations of platelets and fibrinogen in a blood sample on the amplitude were examined.

[Definition of Amplitude]

Fig. 26 is a diagram showing an example of time-dependent changes in the real part of the complex permittivity accompanying blood coagulation. Taking the measured value at a certain time as A and the minimum measured value as B, A was divided by B (Min standard), then 1 was subtracted from the quotient (taking the Min measured value as 0), and the difference was multiplied by 100 (correction due to the value being small); the resulting product was defined as the amplitude CF (the following Mathematical Formula 8).

[Mathematical Formula 8]

$$CF = (A/B - 1) \times 100$$

[Assumption of Contribution Model of Effective Concentrations of Platelets and Fibrinogen to Amplitude]

Considering the facts that in the case where fibrinogen is not present in a blood sample or in the case where fibrinogen in a blood sample does not function, there is no amplitude CF, and that in the case where platelets are not present in a blood sample or in the case where platelets in a blood sample do not function, the amplitude CF depends only on the effective concentration of fibrinogen FIB, a model of the following Mathematical Formula 9 was assumed. Incidentally, a and b are constants, PLT is the effective concentration of platelets, and FIB is the effective concentration of fibrinogen.

1

[Mathematical Formula 9]

$$CF = a \times FIB + b \times (FIB \times PLT)$$

[Definition of Assay Time]

In a blood sample with the addition of Pefabloc, there is a possibility that fibrinogen is continuously affected by Pefabloc. Accordingly, the assay time was defined from a blood sample without the addition of Pefabloc. Specifically, from the measurement results of blood samples without the addition of Pefabloc, 10 minutes, when the clotting time was determined in most of the data, was defined as the assay time (Fig. 27).

[Examination of Model (1)]

In the case where the function of platelets is suppressed with CyD, the amplitude CF varies depending on the effective concentration of fibrinogen FIB. Accordingly, the relation between the amplitude CF of a blood sample having the function of platelets suppressed with CyD and the effective concentration of fibrinogen FIB was examined. Fig. 28 shows the relation between the amplitude CF of a blood sample having the function of platelets completely suppressed with CyD and the effective concentration of fibrinogen FIB.

As shown in Fig. 28, it was confirmed that the following Mathematical Formula 10 holds between the amplitude CF of a blood sample having the function of platelets completely suppressed and the effective concentration of fibrinogen FIB. In addition, a was calculated from Fig. 28 (a = 0.0258).

[Mathematical Formula 10]

$$CF = a \times FIB$$

In addition, from the fact that Mathematical Formula 9 turns into the following Mathematical Formula 11, the relation between the amplitude CF - a x the effective concentration of fibrinogen FIB and the effective concentration of platelets PLT was examined. Examples of the relation between the amplitude CF - a x the effective concentration of fibrinogen FIB and the effective concentration of platelets PLT are shown in Fig. 29 (Example 1) and Fig. 30 (Example 2).

[Mathematical Formula 11]

$$CF - a \times FIB = b \times (FIB \times PLT)$$

Incidentally, CyD suppresses the function of platelets, but does not reduce the number of platelets itself. Accordingly, as measured using a multiparameter automated hematology analyzer (trade name: "pocH" (registered trademark), manufactured by Sysmex Corporation), the number of platelets of a blood sample with the addition of CyD is the same as the number of platelets of a blood sample without the addition of CyD. Therefore, the number of actually functioning platelets was unknown. Thus, the number of functioning platelets was calculated from the CyD concentration by the following method.

A graph was prepared by plotting the CyD concentration on the horizontal axis and, on the vertical axis, a value obtained by subtracting the amplitude of a blood sample having the function of platelets completely suppressed with CyD (CF-CyDY) from the amplitude at each CyD concentration (CF-CyDX) (Fig. 31). Next, using the value of the CyD concentration allowing a straight line to be drawn from the CyD concentration 0, an approximate straight line was drawn. Then, from the equation of the approximate straight line, the CyD concentration at which (CF-CyDX) - (CF-CyDY) = 0 was determined. Supposing that there would be no functioning platelets at the determined CyD concentration, the number of functioning platelets at each CyD concentration was determined from the following Mathematical Formula 12. Incidentally, the number of platelets at a CyD concentration of 0 was calculated from the average number of platelets at various CyD concentrations measured by the multiparameter automated hematology analyzer. A is the number of platelets at a concentration at which there are no functioning platelets, while B is the average number of platelets at various CyD concentrations.

[Mathematical Formula 12]

$$\text{The number of functioning platelets at each CyD concentration} = B - (B/A \times \text{CyD concentration})$$

As shown in Fig. 29 and Fig. 30, it turned out that the inclination varies depending on the concentration of Pefabloc. In addition, if the model holds, then the inclination should be b x the effective concentration of fibrinogen FIB. Accordingly, the relation between the inclination at each Pefabloc concentration and the effective concentration of fibrinogen measured by a fibrinogen measurement device (trade name: "DRIHemato" (registered trademark), manufactured by A&T) was examined (Fig. 32).

As shown in Fig. 32, a correlation is seen between the inclination and the effective concentration of fibrinogen, and thus it turned out that the following Mathematical Formula 13 holds.

[Mathematical Formula 13]

$$\text{Inclination at each Pefabloc concentration} = b \times FIB$$

Therefore, Mathematical Formula 11 holds, and thus it was confirmed that the model holds. In addition, b was calculated from Fig. 32 (b = 0.0014).

[Contribution Proportion of Each Component of Model Equation 9 to Amplitude]

The proportion of the contribution of each component of the above Mathematical Formula 9 (a x the effective concentration of fibrinogen FIB, and b x the effective concentration of fibrinogen FIB x the effective concentration of platelets PLT) to the amplitude CF was calculated. The calculated results are shown in the following Table 2. Incidentally, the effective concentration of fibrinogen was calculated from the amplitude CF using the value of a determined from Fig. 28 described above.

[Table 2]

| | | Experimental Example 1 | | Experimental Example 2 | |
|---|---|---|---|---|---|
| | | a x FIB | b x FIB x PLT | a x FIB | b x FIB x PLT |
| Pefabloc concentration 0 | CyD0 | 55.90% | 44.10% | 54.00% | 46.00% |
| | CyD6 | 76.00% | 24.00% | 83.50% | 16.50% |
| Pefabloc concentration 10 | CyD0 | 55.90% | 44.10% | 54.00% | 46.00% |
| | CyD6 | 100.00% | 0.00% | 100.00% | 0.00% |

As shown in Table 2, it turned out that the contribution of fibrinogen to the amplitude CF was greater than that of platelets.

[Examination of Model (2)]

<Case of Using Effective Concentration of Fibrinogen Measured by Fibrinogen Measurement Device>

The correlation between the amplitude CF calculated using the above Mathematical Formula 9 from the effective concentration of fibrinogen measured by a fibrinogen measurement device (trade name: "DRIHemato" (registered trademark), manufactured by A&T) and the number of platelets measured using a multiparameter automated hematology analyzer (trade name: "pocH" (registered trademark), manufactured by Sysmex Corporation) and the actual measured amplitude CF was examined. The results are shown in Fig. 33.
As shown in Fig. 33, it was confirmed that there was a correlation between the amplitude CF calculated using the above Mathematical Formula 9 and the actual measured amplitude CF.

<Case of Using Effective Concentration of Fibrinogen Calculated from Fig. 28>

The correlation between the amplitude CF calculated using the above Mathematical Formula 9 from the effective concentration of fibrinogen calculated from Fig. 28 and the number of platelets measured using a multiparameter automated hematology analyzer (trade name: "pocH" (registered trademark), manufactured by Sysmex Corporation) and the actual measured amplitude CF was examined. The results are shown in Fig. 34.
As shown in Fig. 34, also in the case of using the effective concentration of fibrinogen calculated from Fig. 28, there is a clear correlation between the amplitude CF calculated using the above Mathematical Formula 9 and the actual measured amplitude CF. Thus, it was confirmed that the above model holds.

Example 3

The effective concentration of fibrinogen measured by a fibrinogen measurement device depends on the hematocrit. Therefore, in order to obtain the results of Example 2 more accurately, the influence of the hematocrit should be taken into consideration. Thus, in the Examination of Model (1) and (2) of Example 2, correction was performed using the following Mathematical Formula 14.

[Mathematical Formula 14]

$$\mathrm{FIB} = \text{fibrinogen concentration (measured by fibrinogen measurement device)} \times (50/(100 - \text{hematocrit}))$$

The hematocrit can be measured using a multiparameter automated hematology analyzer or the like, or can also be calculated from the initial value in the measurement of blood coagulation using electrical characteristics. Specifically, for example, in the case where the complex permittivity of a blood sample is measured, the hematocrit can be determined from its initial value. The relation between the initial value of the complex permittivity and the hematocrit is shown in Fig. 35.
Using the hematocrit measured by the multiparameter automated hematology analyzer, the coefficients in the above Mathematical Formula 9 were as follows: a = 0.0299, b = 0.0016.
In addition, using the hematocrit calculated from the initial value of the complex permittivity, the coefficients in the above Mathematical Formula 9 were as follows: a = 0.0304, b = 0.0017.
Further, the amplitude CF calculated using Mathematical Formula 9 was compared with the actual measured amplitude CF. Fig. 36 and Fig. 37 show the comparison data corresponding to Fig. 33 and Fig. 34, respectively, corrected using the hematocrit measured by the multiparameter automated hematology analyzer.
In addition, Fig. 38 and Fig. 39 show the comparison data corresponding to Fig. 33 and Fig. 34, respectively, corrected using the hematocrit calculated from the initial value of the complex permittivity.
As shown in Fig. 36 to Fig. 39, similarly to Fig. 33 and Fig. 34, there were correlations. In addition, Fig. 36 to Fig. 39 showed better correlations as compared with the corresponding correlations in Fig. 33 and Fig. 34. Accordingly, correction using the hematocrit was turned out to be effective.
Incidentally, the present disclosure is not limited to the above models, and may be suitably set. For example, the form of the interference term between platelets and fibrinogen may be changed, or the effectiveness of PFG and CyD may be incorporated.
1: Blood condition analysis system

10: Electrical characteristic measurement device
11: Blood condition analysis device
12: Server
13: Display device
14: Network

## Claims

**1.** A blood condition analysis device, comprising at least an analysis unit for evaluating, with respect to two or more blood samples adjusted from one blood specimen and having different drug types or concentrations, the influence of the drug or a factor in the blood on the coagulation system or fibrinolysis system of the blood utilizing data of time-dependent changes in electrical characteristics measured at a specific frequency or frequency band.

**2.** The blood condition analysis device according to claim 1, wherein the drug is an activator or suppressor of the coagulation system or fibrinolysis system of the blood.

**3.** The blood condition analysis device according to claim 1, wherein the analysis unit calculates, from data of time-dependent changes in the electrical characteristics of each blood sample, at least one kind of value selected from the group consisting of the coagulation time, the clot formation time, the maximum clot firmness, the maximum lysis, the coagulation amplitude, the coagulation rate, the clot firmness after a predetermined period of time from the coagulation time, the clot firmness after a predetermined period of time from the maximum firmness, and the clot linear velocity after the maximum firmness, and performs the evaluation on the basis of the calculated value.

**4.** The blood condition analysis device according to claim 3, wherein the analysis unit corrects the calculated value with the hematocrit of the blood sample.

**5.** The blood condition analysis device according to claim 1, wherein the analysis unit evaluates one or both of the influence of platelet factors and the influence of fibrinogen on the coagulation system of the blood.

**6.** The blood condition analysis device according to claim 5, wherein the drug is a platelet function suppressor, a fibrinogen function suppressor, or a fibrin polymerization suppressor.

**7.** The blood condition analysis device according to claim 6, wherein the analysis unit evaluates the influence of the drug on the basis of the difference in data of time-dependent changes in electrical characteristics or the difference in values calculated from the data of time-dependent changes between a blood sample not containing the drug and a blood sample containing the drug at an arbitrary concentration.

**8.** The blood condition analysis device according to claim 6, wherein the analysis unit estimates the minimum drug dose to eliminate the influence of a target factor contained in the blood sample.

**9.** The blood condition analysis device according to claim 1, wherein the analysis unit evaluates the influence of plasmin or plasminogen on the fibrinolysis system of the blood.

**10.** The blood condition analysis device according to claim 9, wherein the drug is an activator or inhibitor of plasminogen or plasmin.

**11.** The blood condition analysis device according to claim 10, wherein the analysis unit evaluates the influence of plasmin or plasminogen by comparing data of time-dependent changes in electrical characteristics between a blood sample not containing the drug and a blood sample containing the drug at an arbitrary concentration.

**12.** The blood condition analysis device according to claim 1, wherein the drug is heparin, and the analysis unit evaluates the suppressing effect of the heparin on blood coagulation.

**13.** A blood condition analysis system, comprising:

an electrical characteristic measurement device including a measurement unit for measuring the electrical characteristics of two or more blood samples adjusted from one blood specimen and having different drug types or concentrations over time at a specific frequency or frequency band; and
a blood condition analysis device including an analysis unit for evaluating the influence of the drug or a factor in the blood on the coagulation system or fibrinolysis system of the blood utilizing data of time-dependent changes in the electrical characteristics measured by the electrical characteristic measurement device.

**14.** The blood condition analysis system according to claim 13, further comprising a server including an information storage unit for storing the measurement data from the electrical characteristic measurement device and/or the analysis results from the blood condition analysis device,
the server being connected to the electrical characteristic measurement device and/or the blood condition analysis device through a network.

**15.** A blood condition analysis method, comprising:

a measurement step of measuring the electrical characteristics of two or more blood samples adjusted from one blood specimen and having different drug types or concentrations over time at a specific frequency or frequency band; and
an analysis step of evaluating the influence of the drug or a factor in the blood on the coagulation system or fibrinolysis system of the blood utilizing data of time-dependent changes in the electrical characteristics measured in the measurement step.

**16.** Aprogram for causing a computer to implement an analysis function of evaluating, with respect to two or more blood samples adjusted from one blood specimen and having different drug types or concentrations, the influence of the drug or a factor in the blood on the coagulation system or fibrinolysis system of the blood utilizing data of time-dependent changes in electrical characteristics measured at a specific frequency or frequency band.

FIG. 1

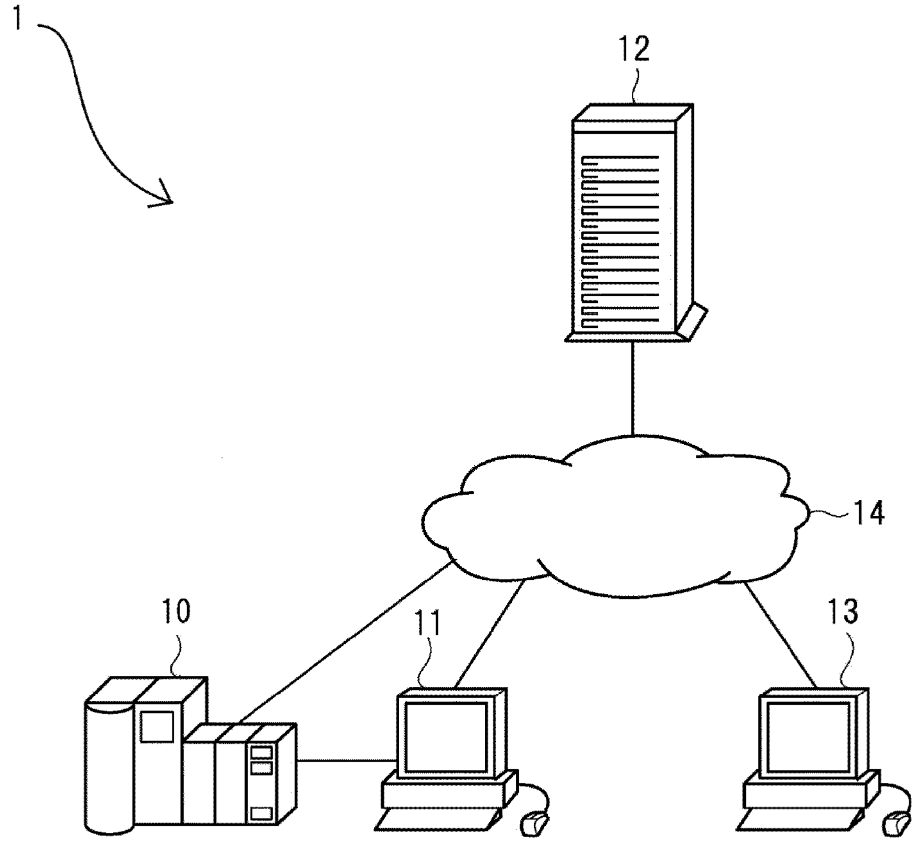

FIG. 2

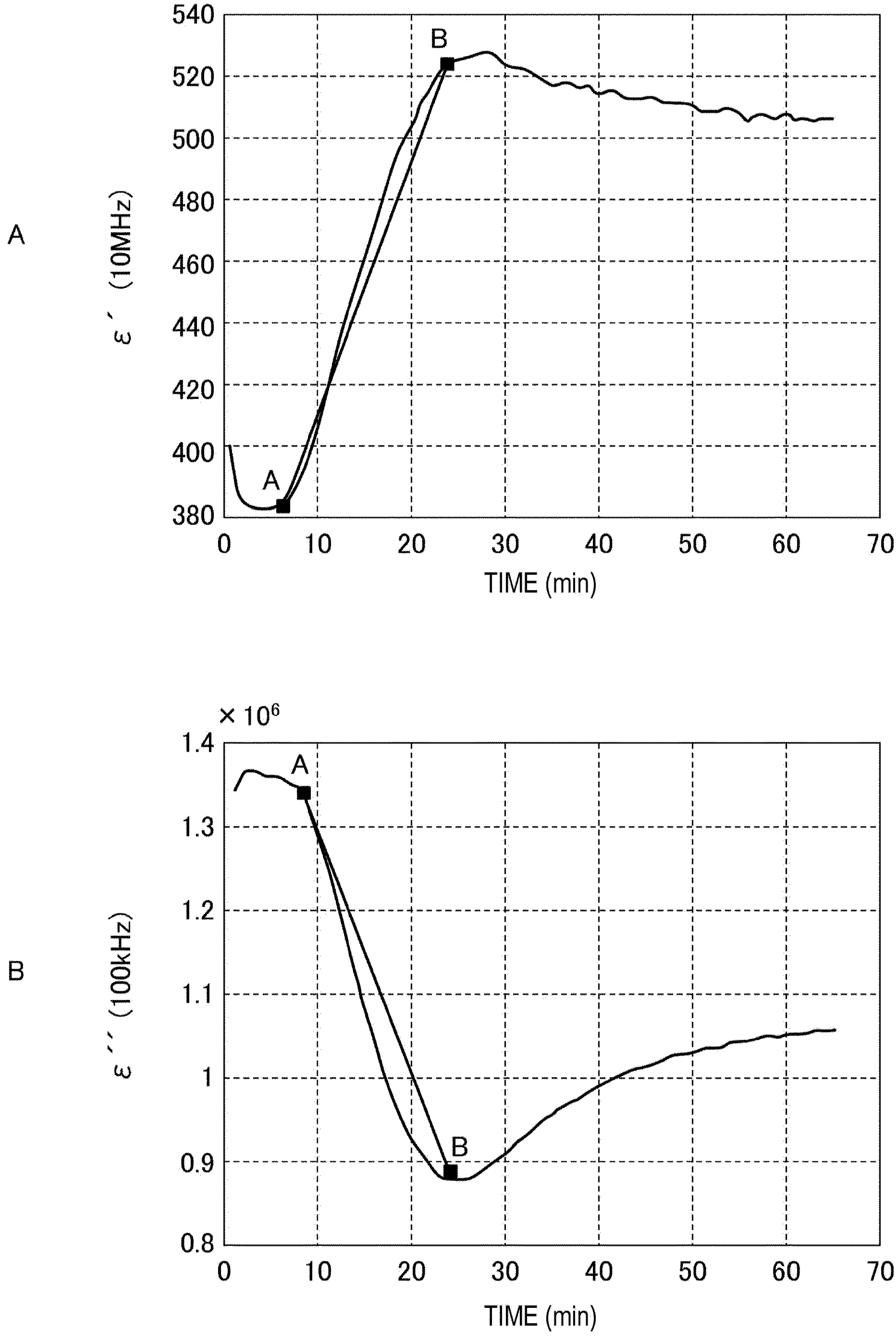
A
ε′ (10MHz)
540
520
500
480
460
440
420
400
380
0
10
20
30
40
50
60
70
TIME (min)
A
B
B
× 10^6
ε′′ (100kHz)
1.4
1.3
1.2
1.1
1
0.9
0.8
0
10
20
30
40
50
60
70
TIME (min)
A
B

FIG. 3

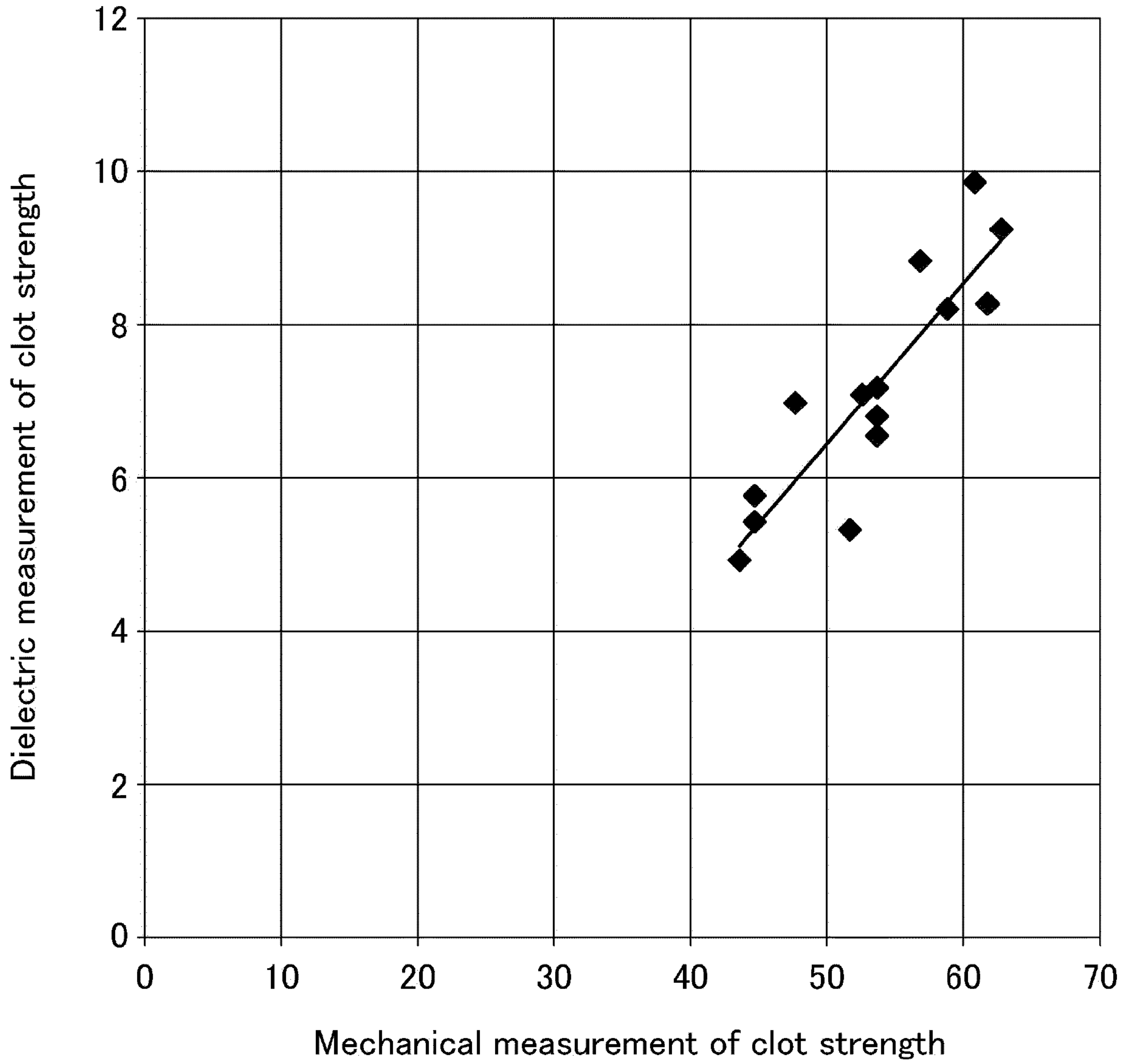
12
10
8
6
4
2
0
0
10
20
30
40
50
60
70
Dielectric measurement of clot strength
Mechanical measurement of clot strength

FIG. 4

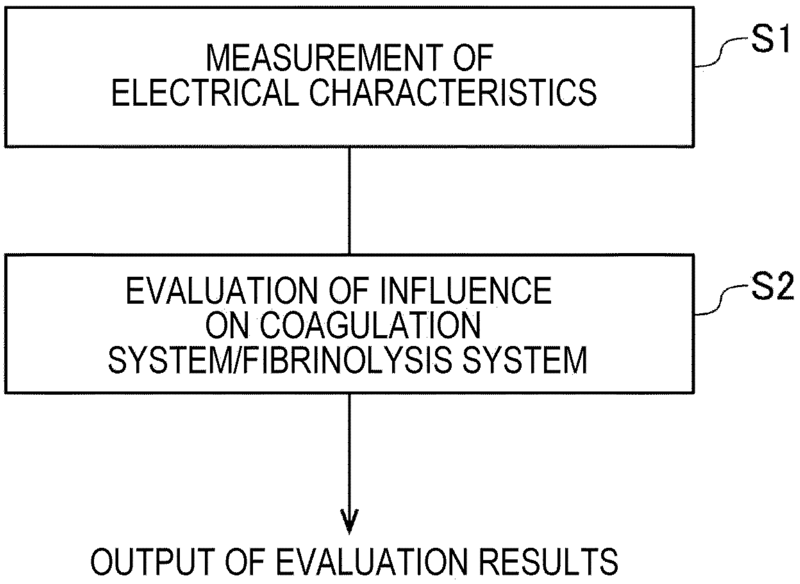
MEASUREMENT OF ELECTRICAL CHARACTERISTICS
S1
EVALUATION OF INFLUENCE ON COAGULATION SYSTEM/FIBRINOLYSIS SYSTEM
S2
OUTPUT OF EVALUATION RESULTS

FIG. 5

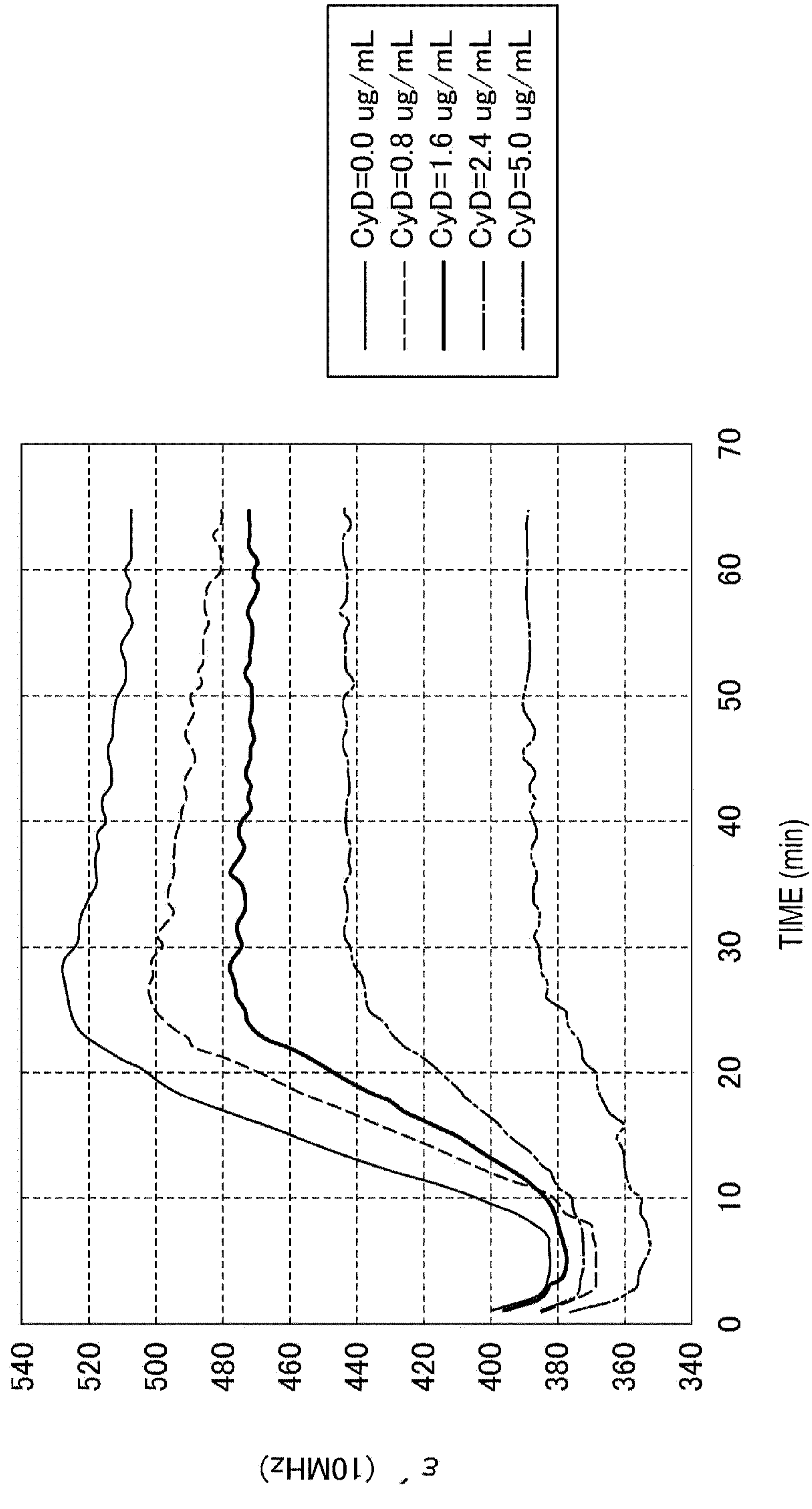
CyD=0.0 ug/mL
CyD=0.8 ug/mL
CyD=1.6 ug/mL
CyD=2.4 ug/mL
CyD=5.0 ug/mL
ε' (10MHz)
540
520
500
480
460
440
420
400
380
360
340
0
10
20
30
40
50
60
70
TIME (min)

FIG. 6

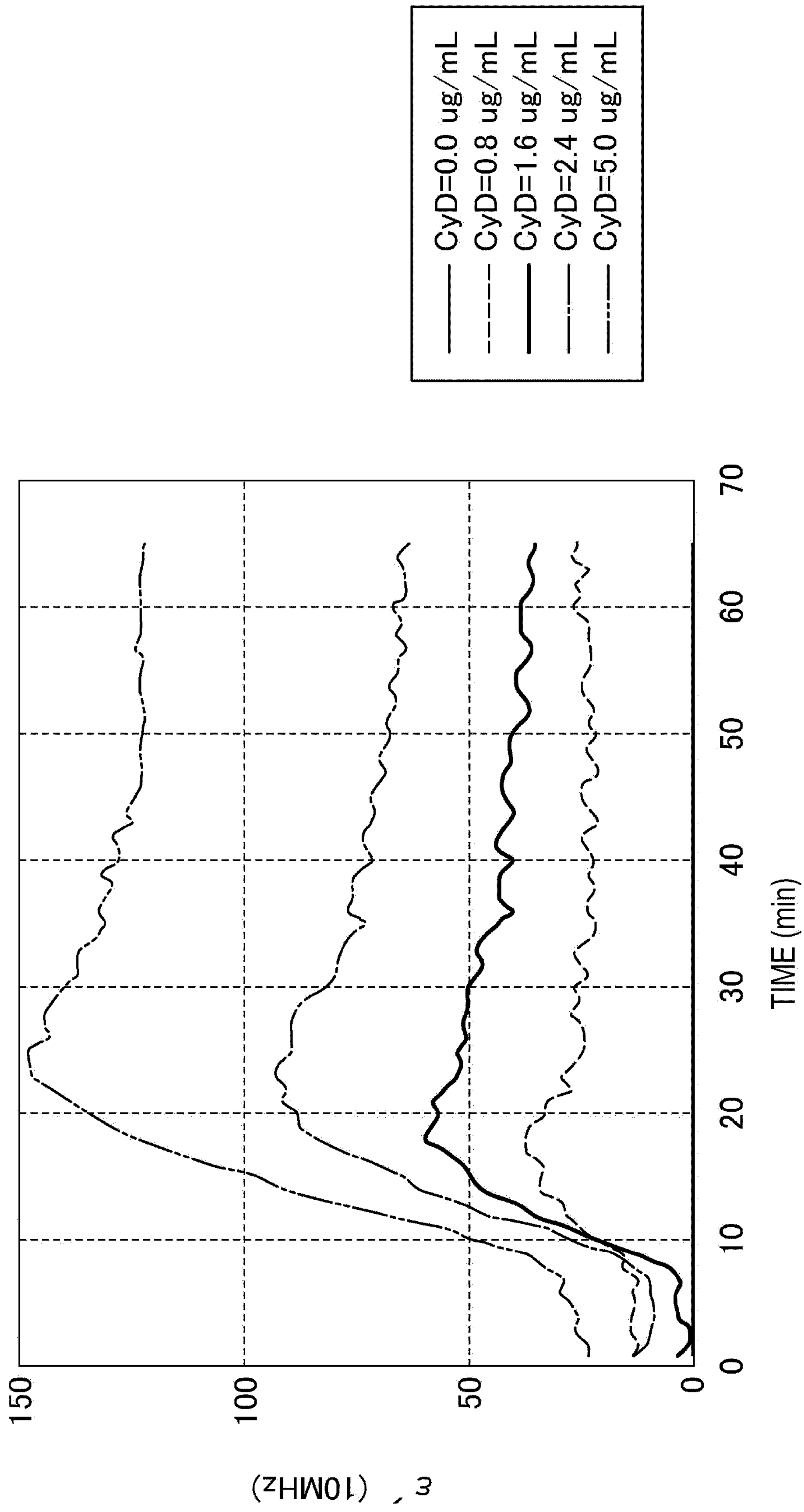
CyD=0.0 ug/mL
CyD=0.8 ug/mL
CyD=1.6 ug/mL
CyD=2.4 ug/mL
CyD=5.0 ug/mL
ε' (10MHz)
0
50
100
150
TIME (min)
0
10
20
30
40
50
60
70

FIG. 7

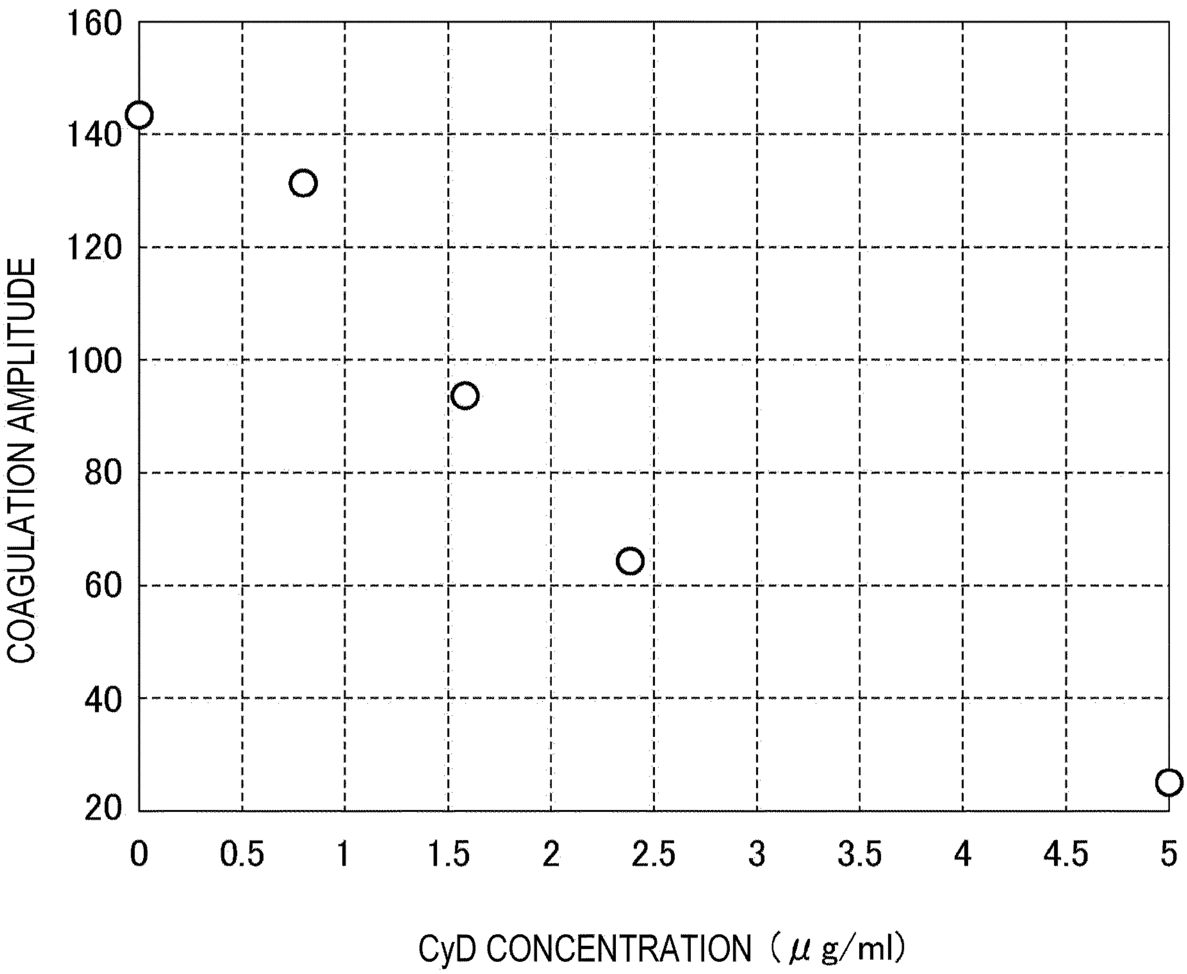
160
140
120
100
80
60
40
20
COAGULATION AMPLITUDE
0
0.5
1
1.5
2
2.5
3
3.5
4
4.5
5
CyD CONCENTRATION (μg/ml)

FIG. 8

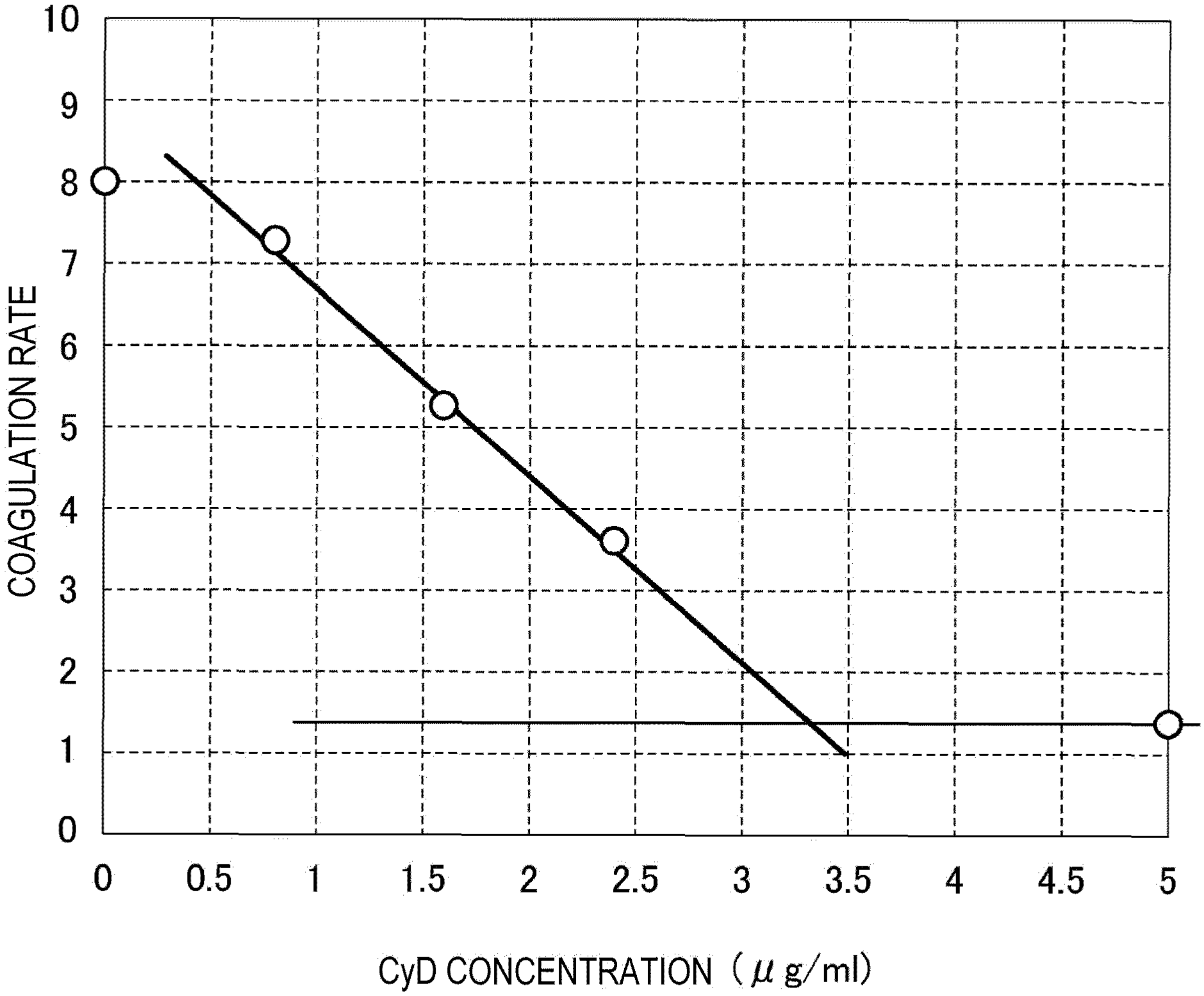
10
9
8
7
6
5
4
3
2
1
0
COAGULATION RATE
0
0.5
1
1.5
2
2.5
3
3.5
4
4.5
5
CyD CONCENTRATION (μg/ml)

# FIG. 9

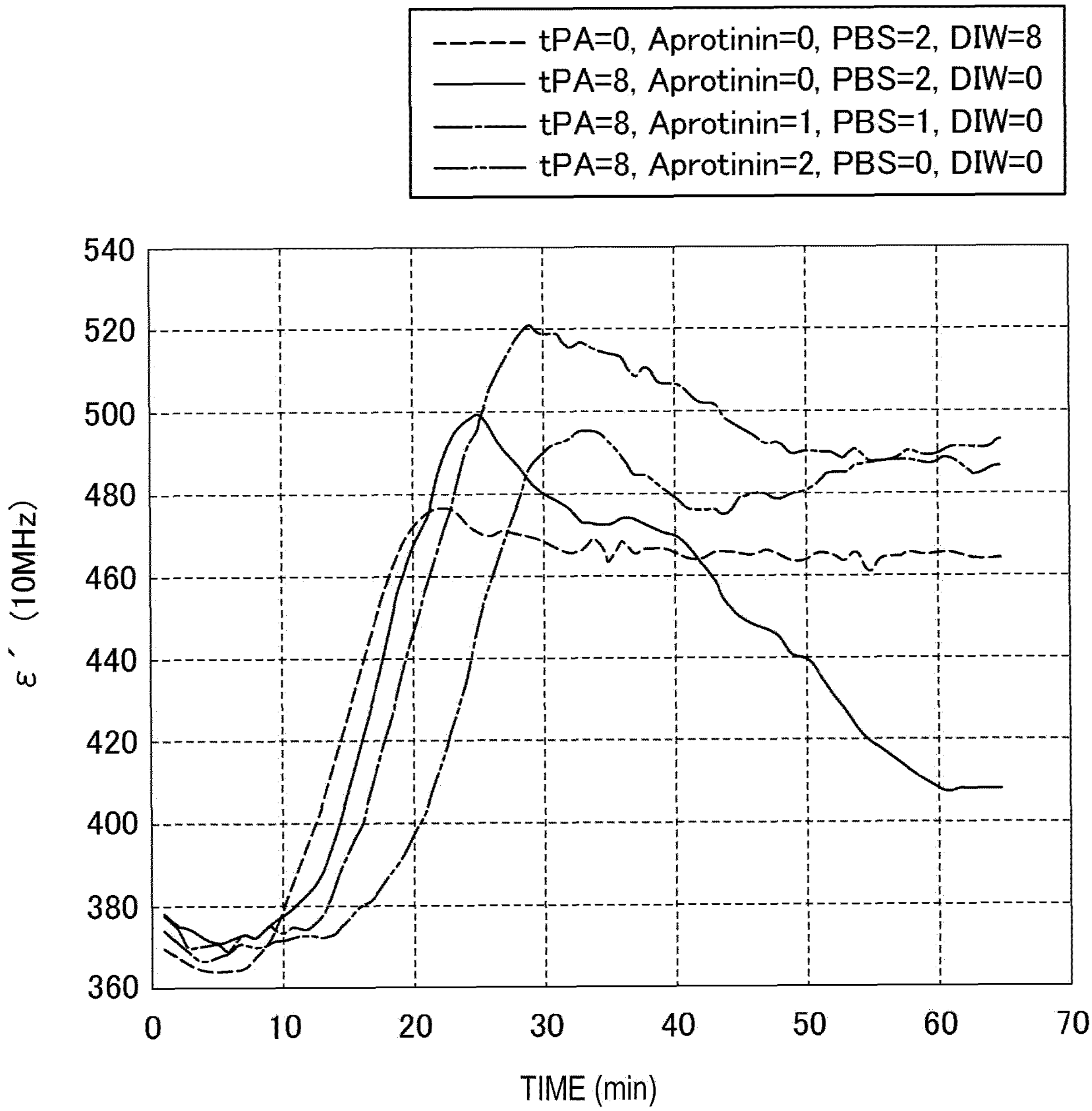
tPA=0, Aprotinin=0, PBS=2, DIW=8
tPA=8, Aprotinin=0, PBS=2, DIW=0
tPA=8, Aprotinin=1, PBS=1, DIW=0
tPA=8, Aprotinin=2, PBS=0, DIW=0
540
520
500
480
460
440
420
400
380
360
ε´ (10MHz)
0
10
20
30
40
50
60
70
TIME (min)

FIG. 10

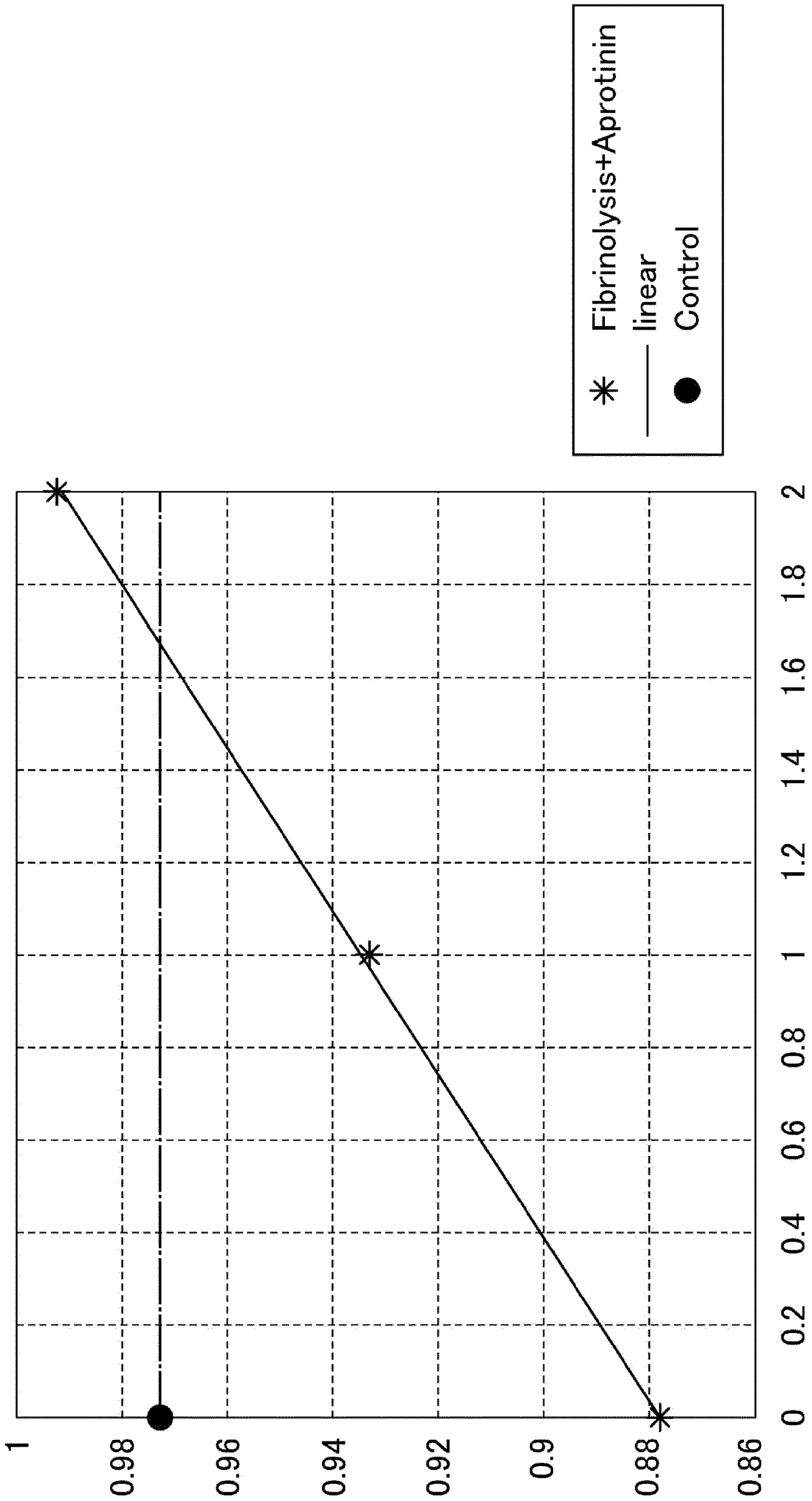
Fibrinolysis+Aprotinin
linear
Control
1
0.98
0.96
0.94
0.92
0.9
0.88
0.86
0
0.2
0.4
0.6
0.8
1
1.2
1.4
1.6
1.8
2

FIG. 11

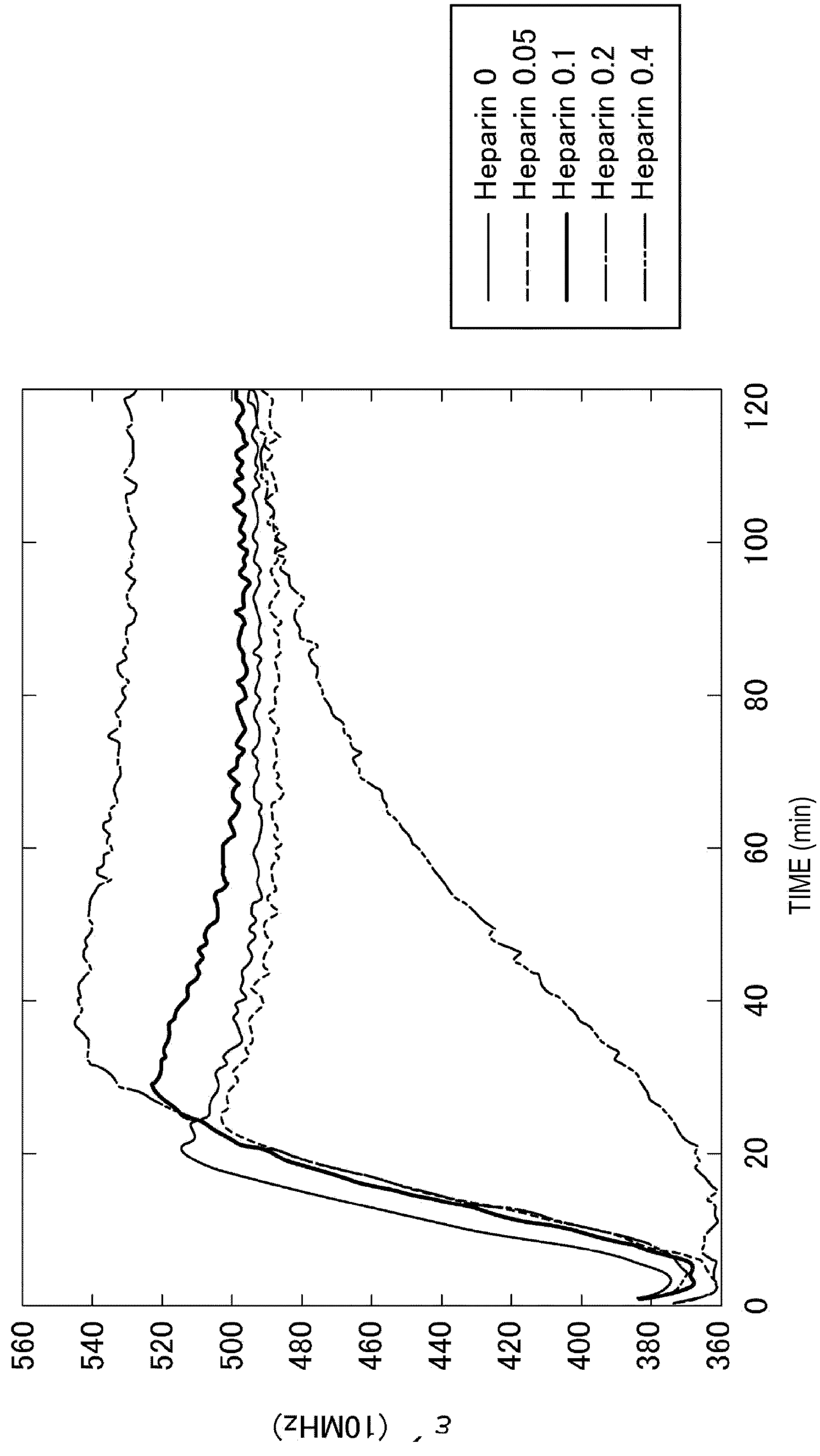
Heparin 0
Heparin 0.05
Heparin 0.1
Heparin 0.2
Heparin 0.4
ε′ (10MHz)
560
540
520
500
480
460
440
420
400
380
360
0
20
40
60
80
100
120
TIME (min)

# FIG. 12

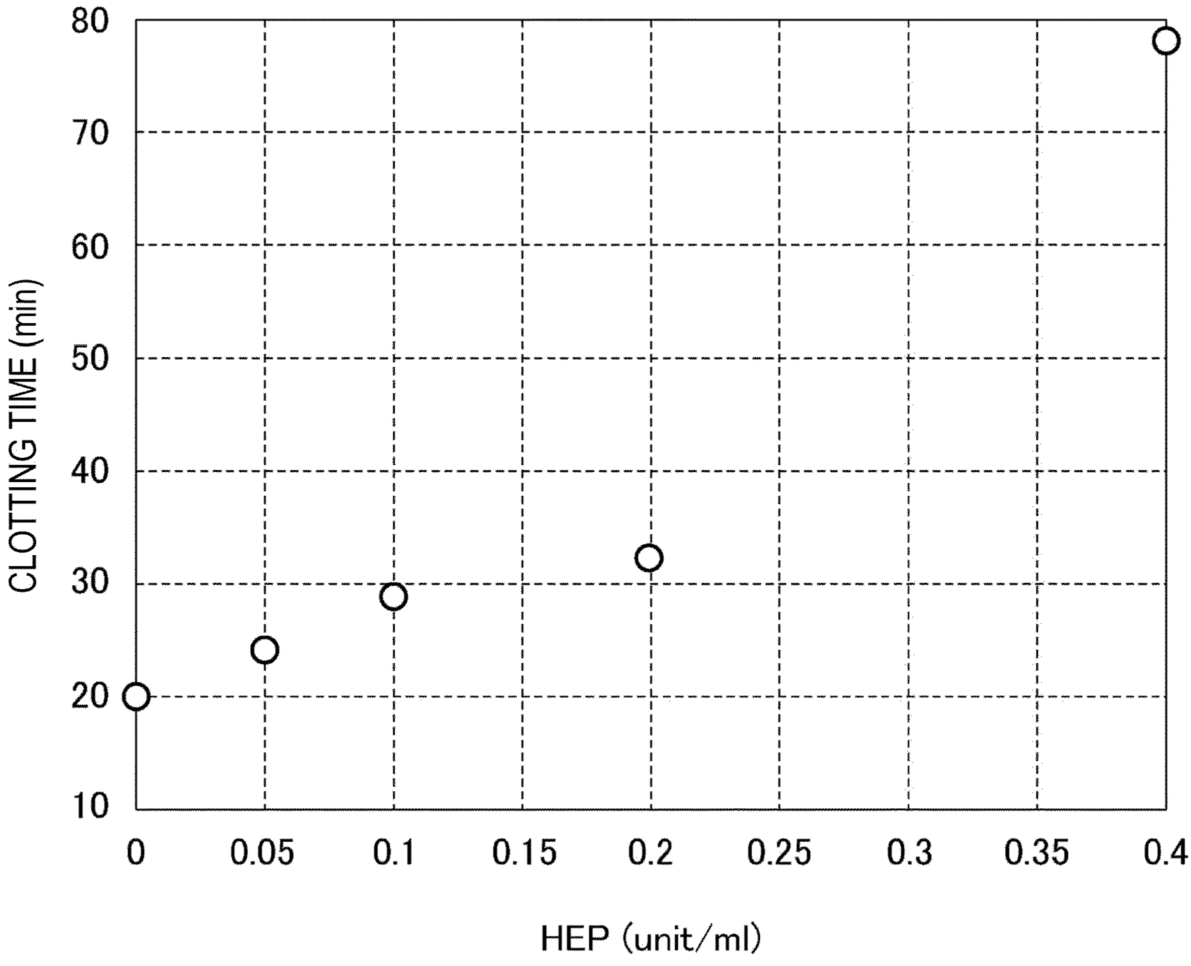
80
70
60
50
40
30
20
10
CLOTTING TIME (min)
0
0.05
0.1
0.15
0.2
0.25
0.3
0.35
0.4
HEP (unit/ml)

FIG. 13

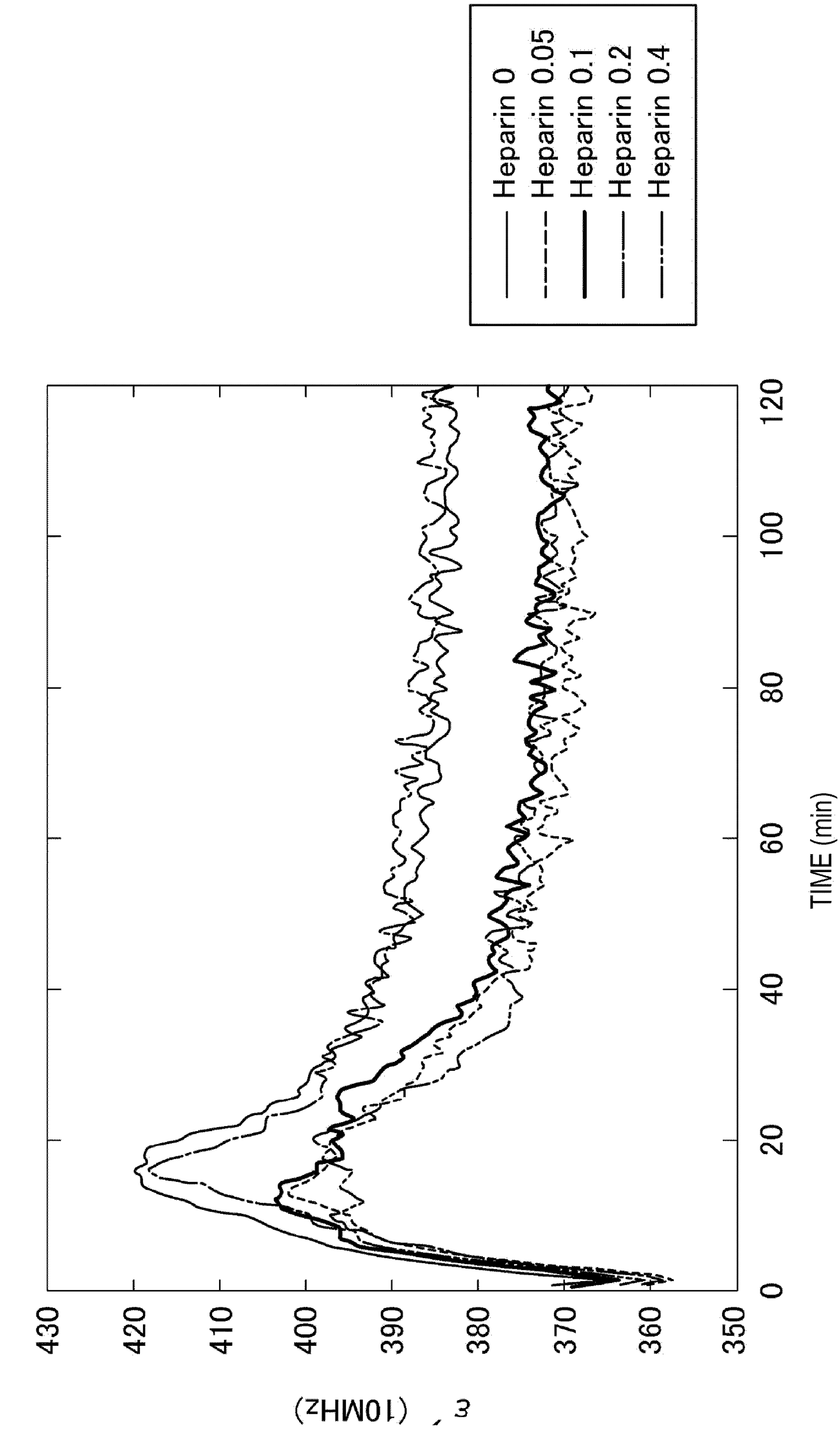
Heparin 0
Heparin 0.05
Heparin 0.1
Heparin 0.2
Heparin 0.4
430
420
410
400
390
380
370
360
350
ε' (10MHz)
0
20
40
60
80
100
120
TIME (min)

*FIG. 14*

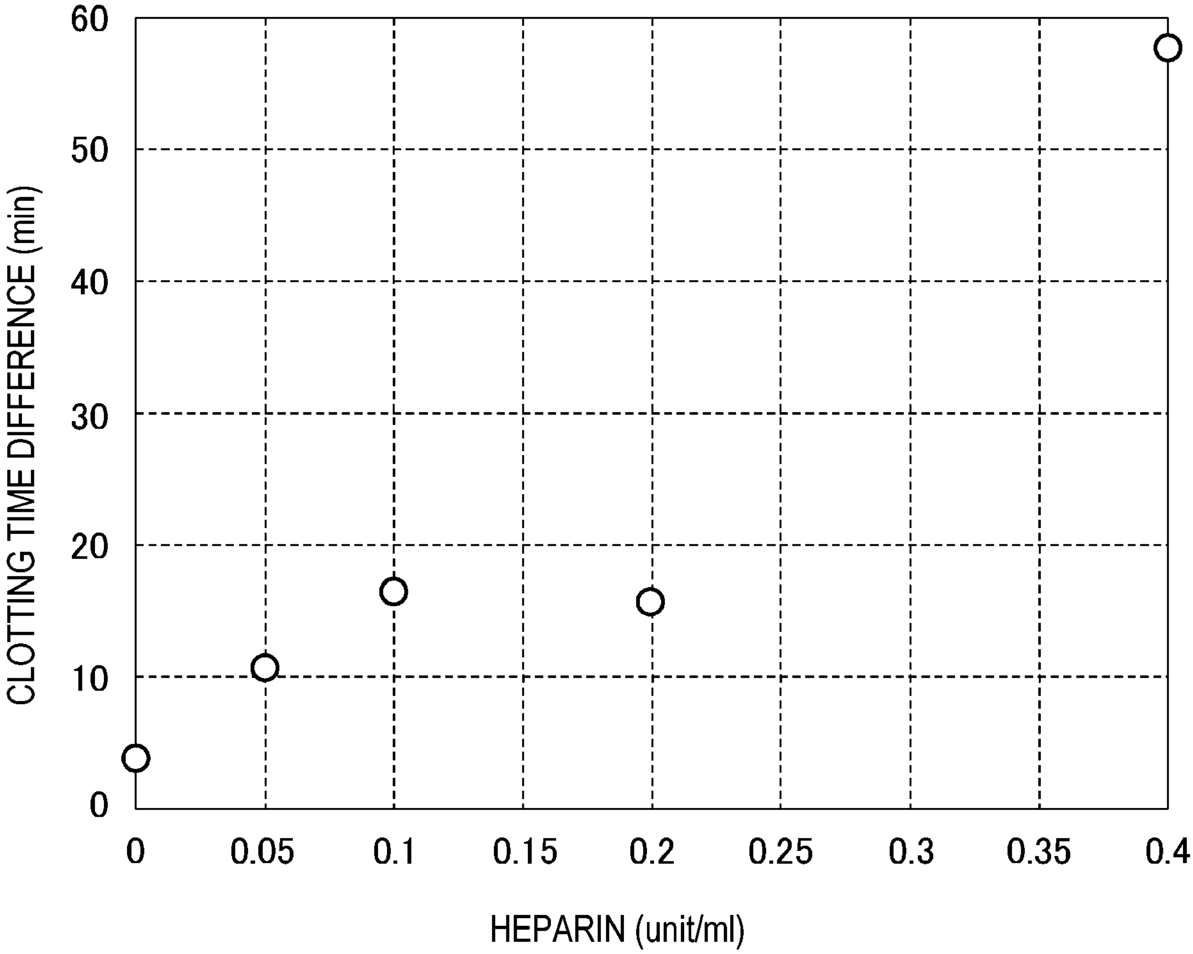

60
50
40
30
20
10
0
CLOTTING TIME DIFFERENCE (min)
0
0.05
0.1
0.15
0.2
0.25
0.3
0.35
0.4
HEPARIN (unit/ml)

# FIG. 15

A

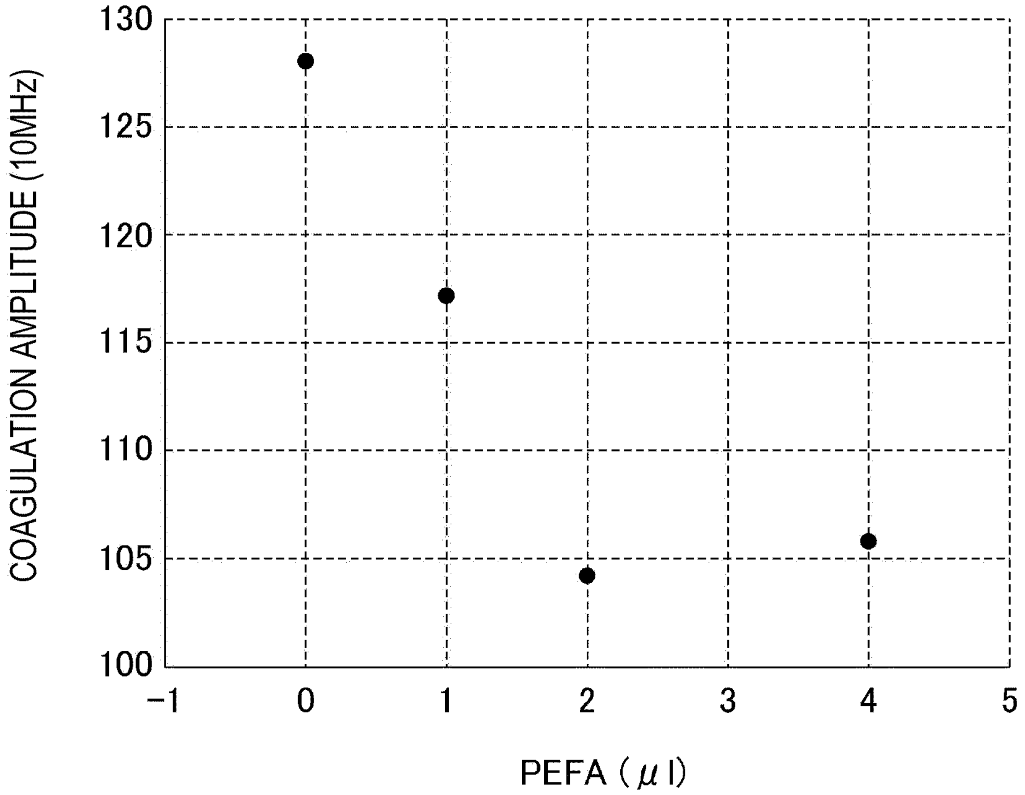
COAGULATION AMPLITUDE (10MHz)
130
125
120
115
110
105
100
−1
0
1
2
3
4
5
PEFA (μl)

B

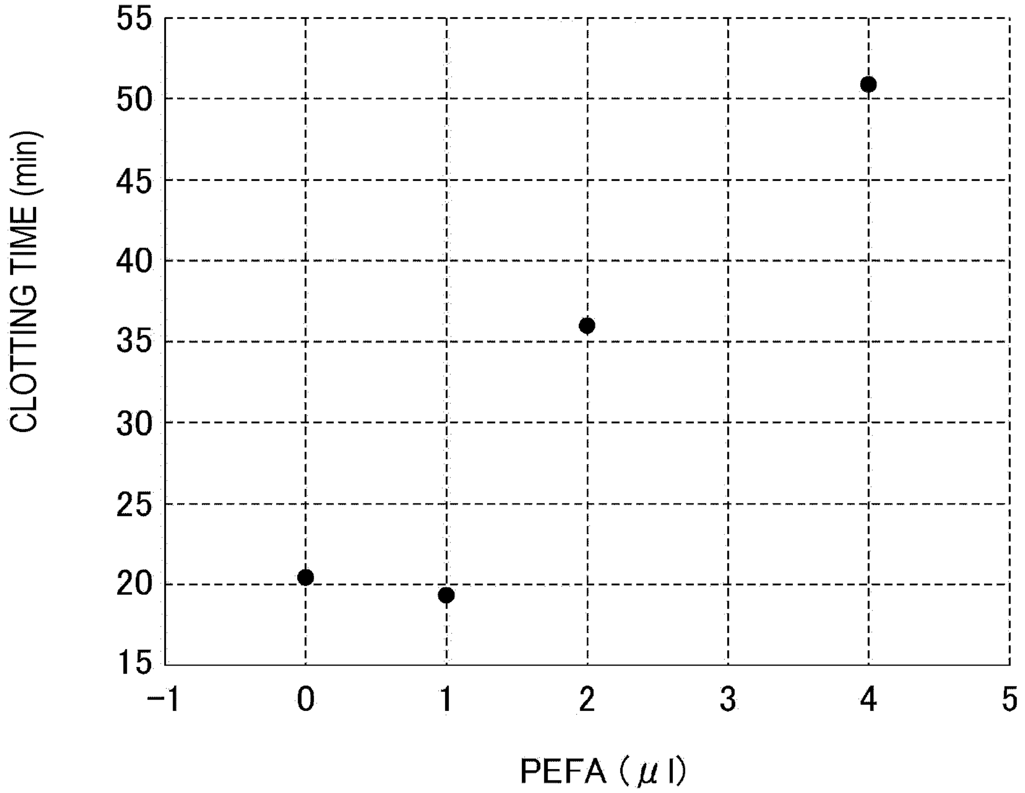
CLOTTING TIME (min)
55
50
45
40
35
30
25
20
15
−1
0
1
2
3
4
5
PEFA (μl)

FIG. 16

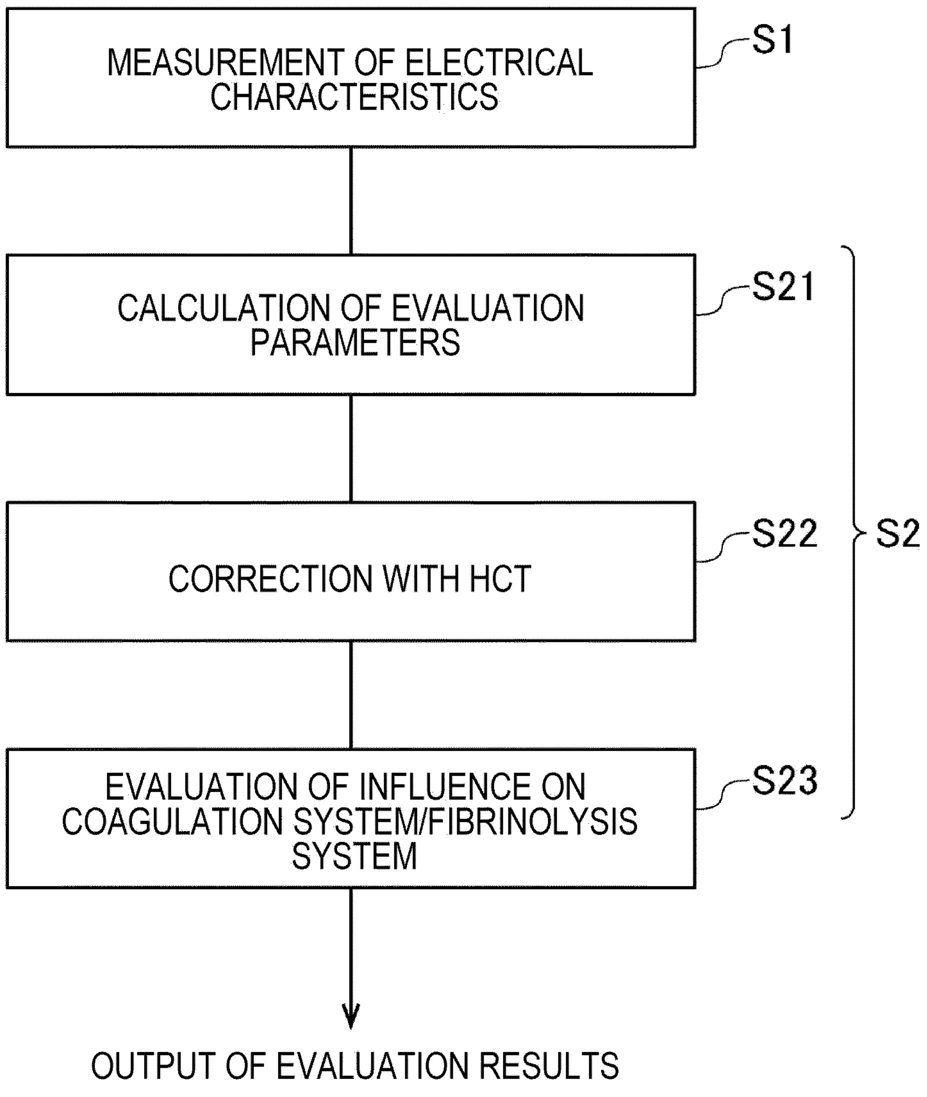
MEASUREMENT OF ELECTRICAL CHARACTERISTICS
S1
CALCULATION OF EVALUATION PARAMETERS
S21
CORRECTION WITH HCT
S22
S2
EVALUATION OF INFLUENCE ON COAGULATION SYSTEM/FIBRINOLYSIS SYSTEM
S23
OUTPUT OF EVALUATION RESULTS

FIG. 17

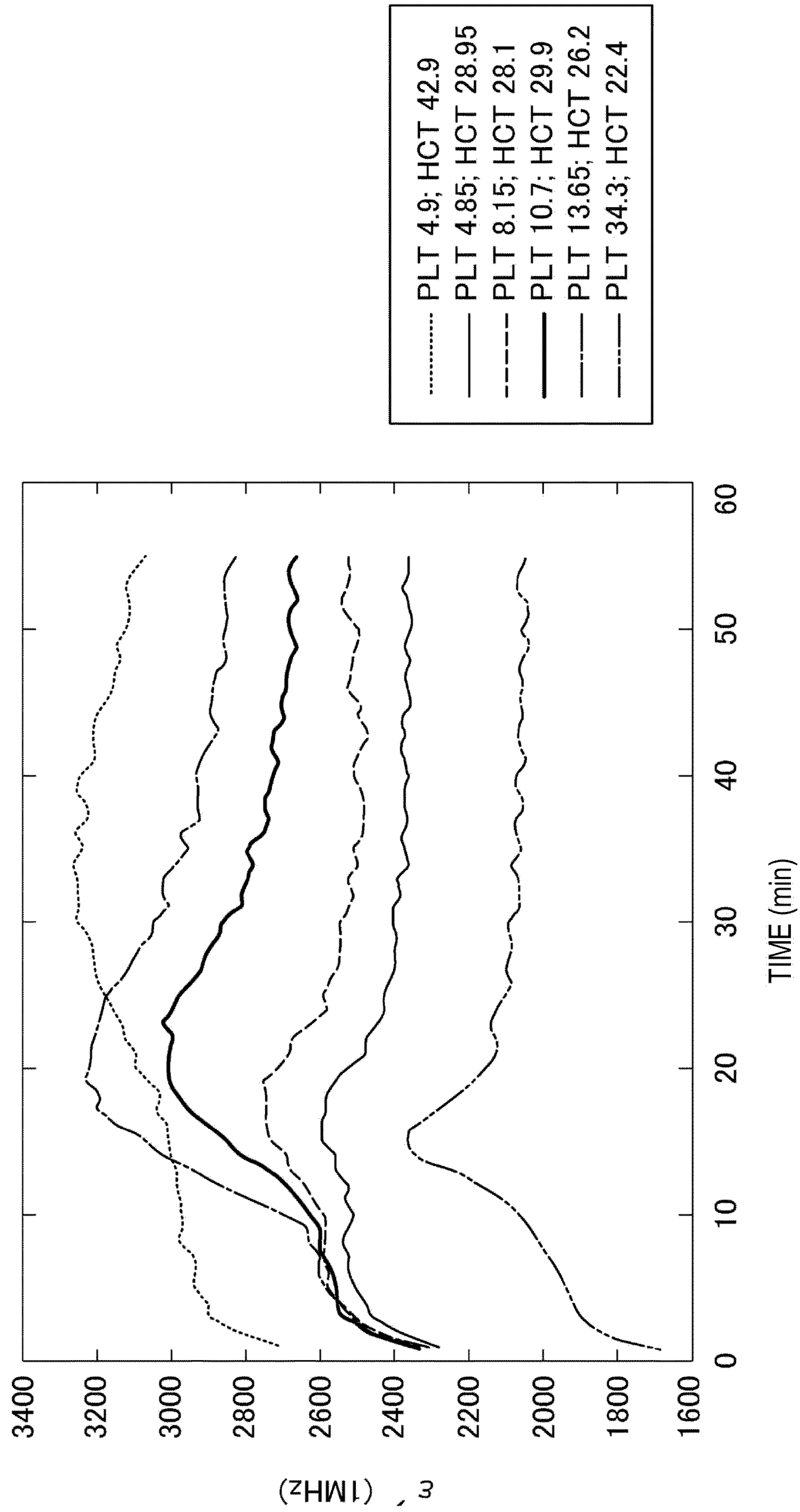
PLT 4.9; HCT 42.9
PLT 4.85; HCT 28.95
PLT 8.15; HCT 28.1
PLT 10.7; HCT 29.9
PLT 13.65; HCT 26.2
PLT 34.3; HCT 22.4
ε′ (1MHz)
3400
3200
3000
2800
2600
2400
2200
2000
1800
1600
0
10
20
30
40
50
60
TIME (min)

FIG. 18

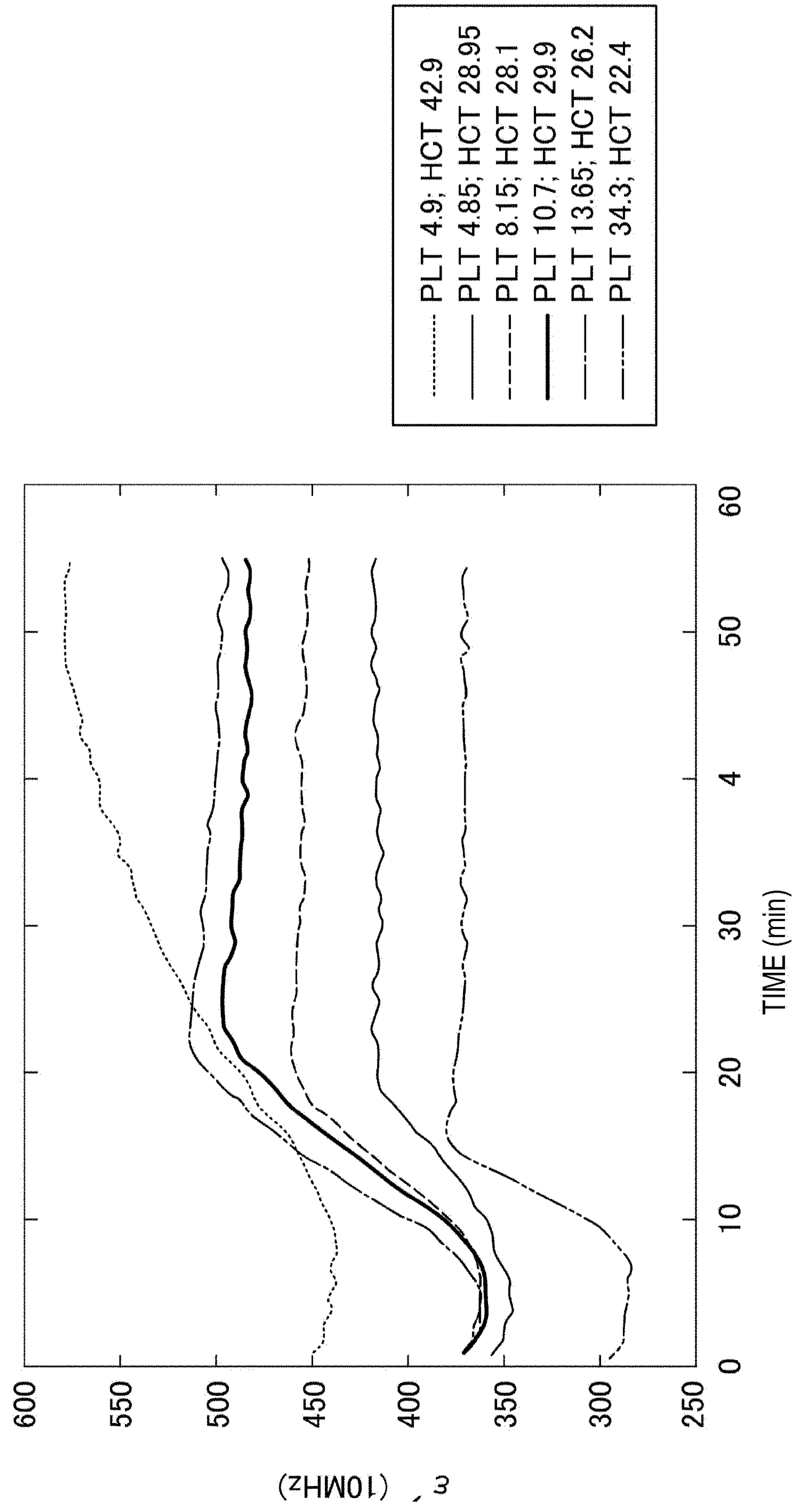
PLT 4.9; HCT 42.9
PLT 4.85; HCT 28.95
PLT 8.15; HCT 28.1
PLT 10.7; HCT 29.9
PLT 13.65; HCT 26.2
PLT 34.3; HCT 22.4
ε' (10MHz)
600
550
500
450
400
350
300
250
0
10
20
30
4
50
60
TIME (min)

FIG. 19

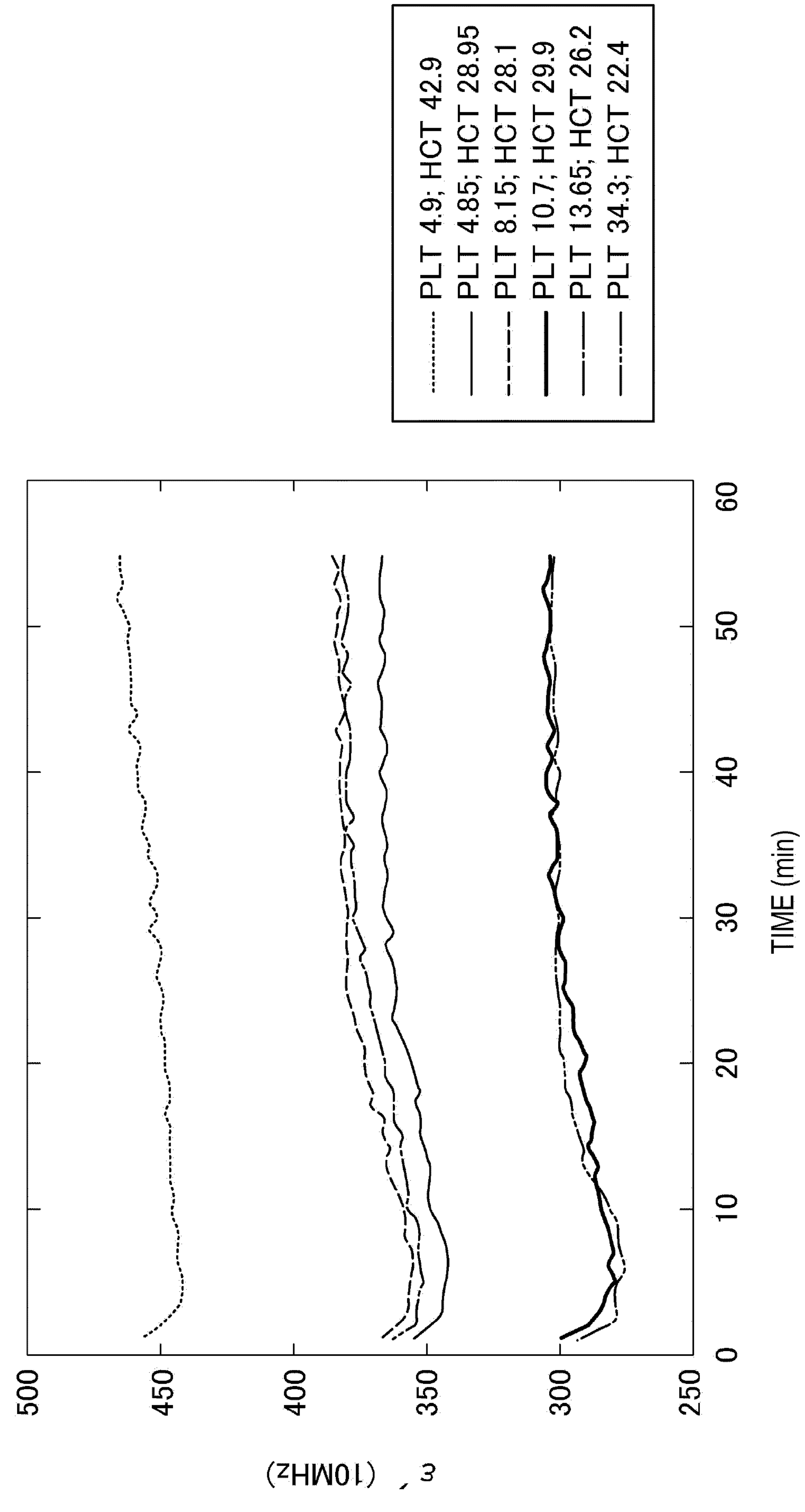
PLT 4.9; HCT 42.9
PLT 4.85; HCT 28.95
PLT 8.15; HCT 28.1
PLT 10.7; HCT 29.9
PLT 13.65; HCT 26.2
PLT 34.3; HCT 22.4
ε' (10MHz)
500
450
400
350
300
250
0
10
20
30
40
50
60
TIME (min)

FIG. 20

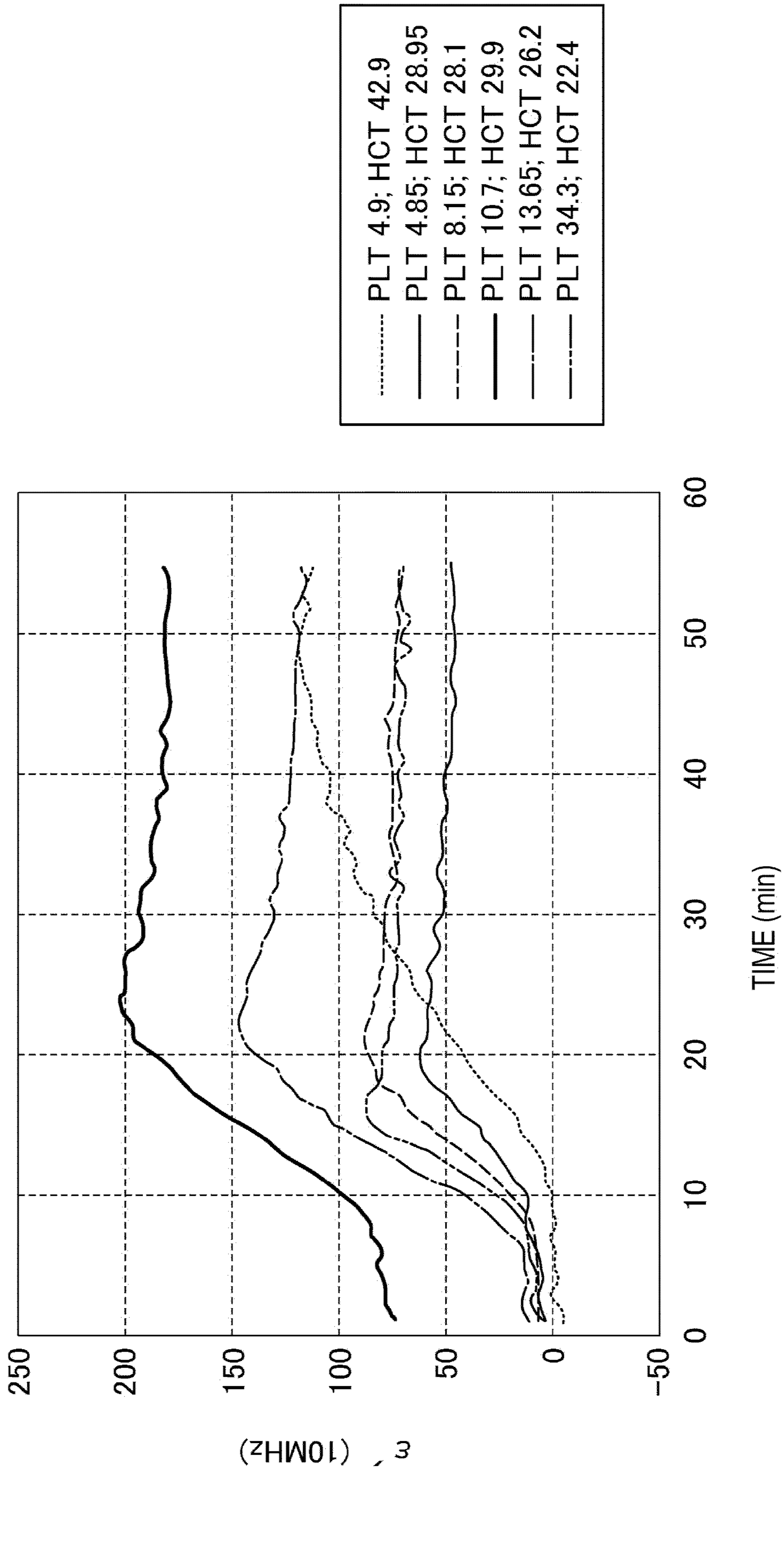
PLT 4.9; HCT 42.9
PLT 4.85; HCT 28.95
PLT 8.15; HCT 28.1
PLT 10.7; HCT 29.9
PLT 13.65; HCT 26.2
PLT 34.3; HCT 22.4
ε' (10MHz)
250
200
150
100
50
0
−50
0
10
20
30
40
50
60
TIME (min)

FIG. 21

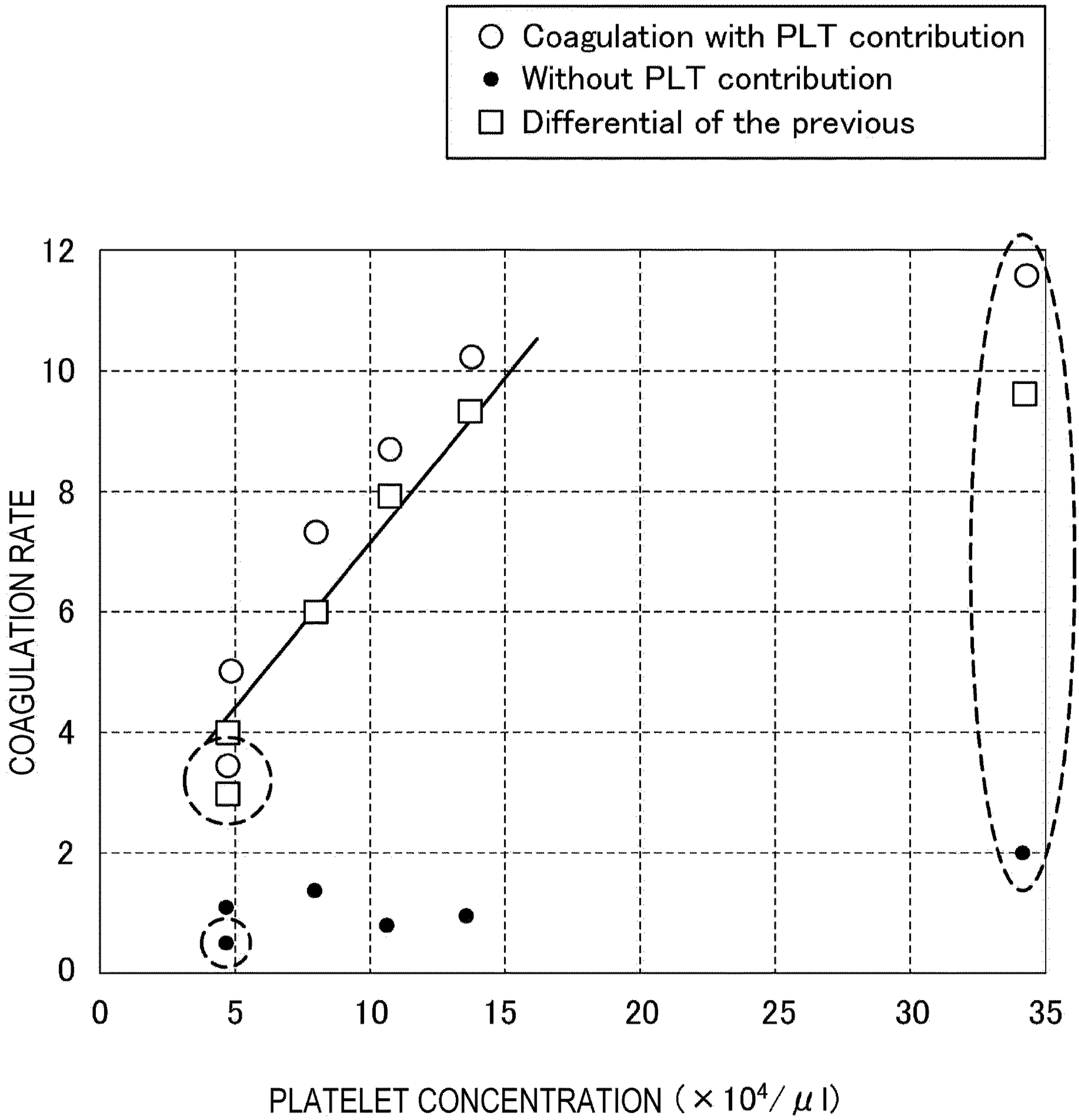
Coagulation with PLT contribution
Without PLT contribution
Differential of the previous
COAGULATION RATE
0
2
4
6
8
10
12
0
5
10
15
20
25
30
35
PLATELET CONCENTRATION (×10⁴/μl)

# FIG. 22

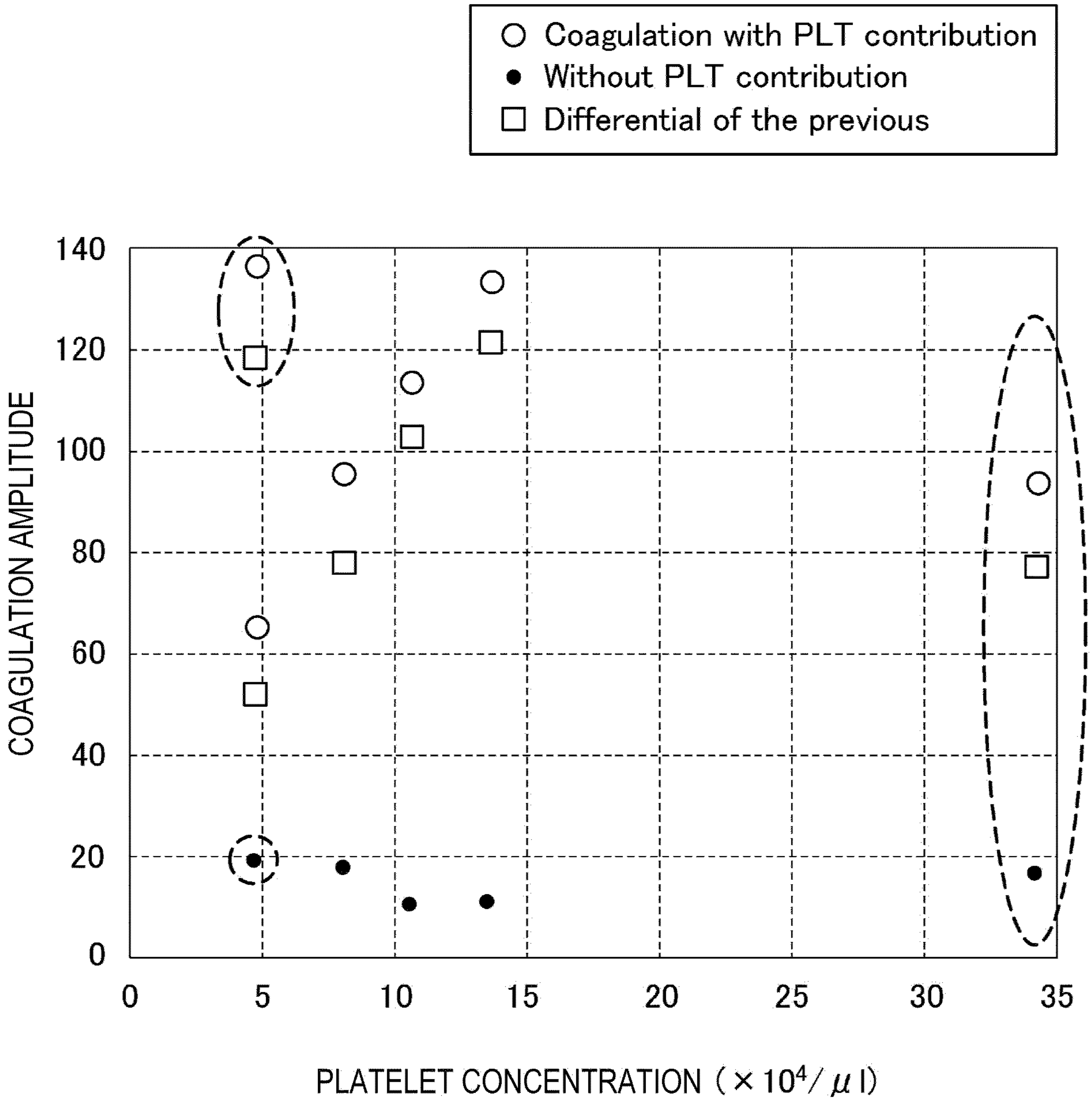
Coagulation with PLT contribution
Without PLT contribution
Differential of the previous
COAGULATION AMPLITUDE
0
20
40
60
80
100
120
140
0
5
10
15
20
25
30
35
PLATELET CONCENTRATION (×10⁴/μl)

FIG. 23

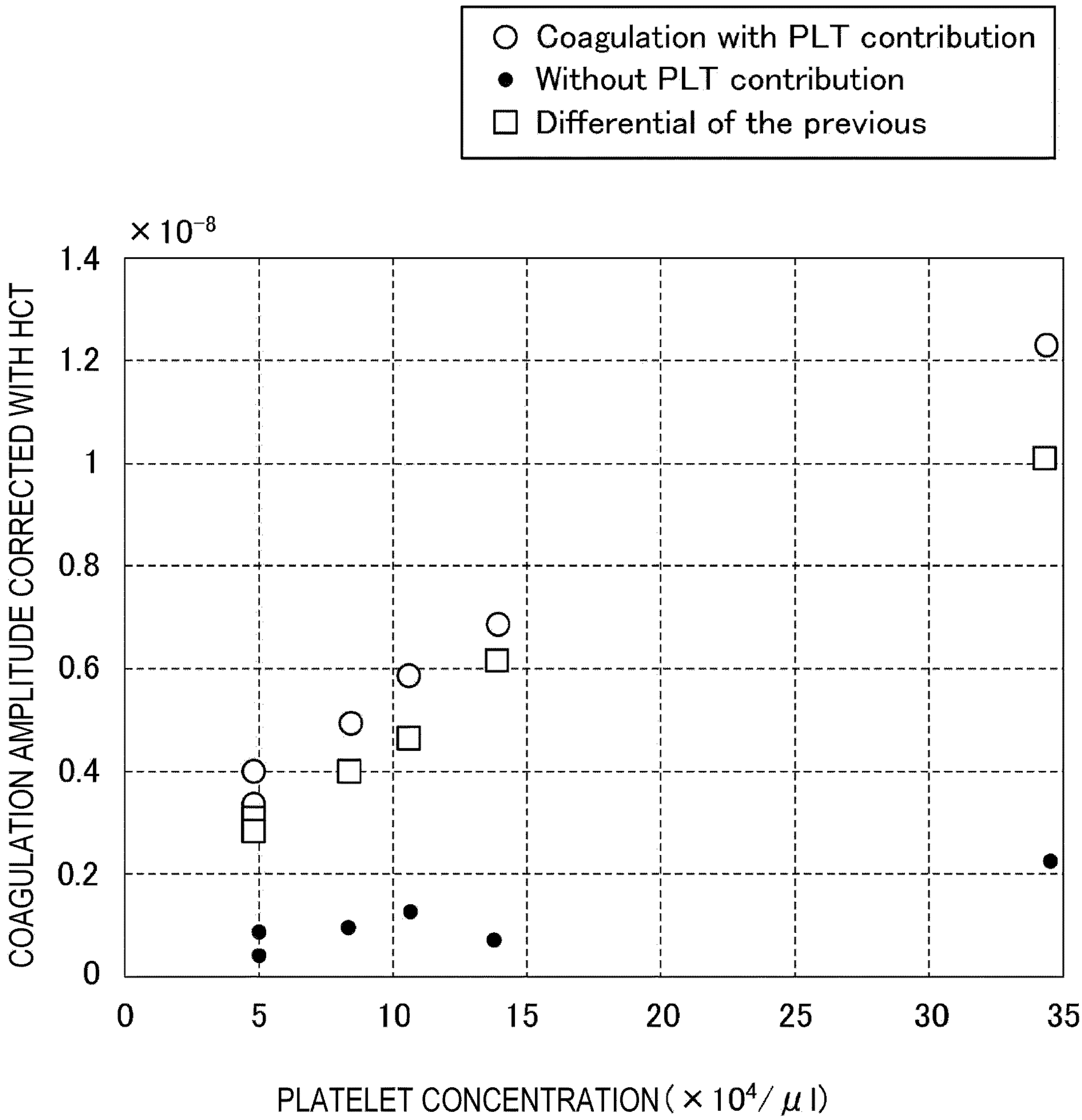
Coagulation with PLT contribution
Without PLT contribution
Differential of the previous
×10⁻⁸
1.4
1.2
1
0.8
0.6
0.4
0.2
0
COAGULATION AMPLITUDE CORRECTED WITH HCT
0
5
10
15
20
25
30
35
PLATELET CONCENTRATION (×10⁴/μl)

FIG. 24

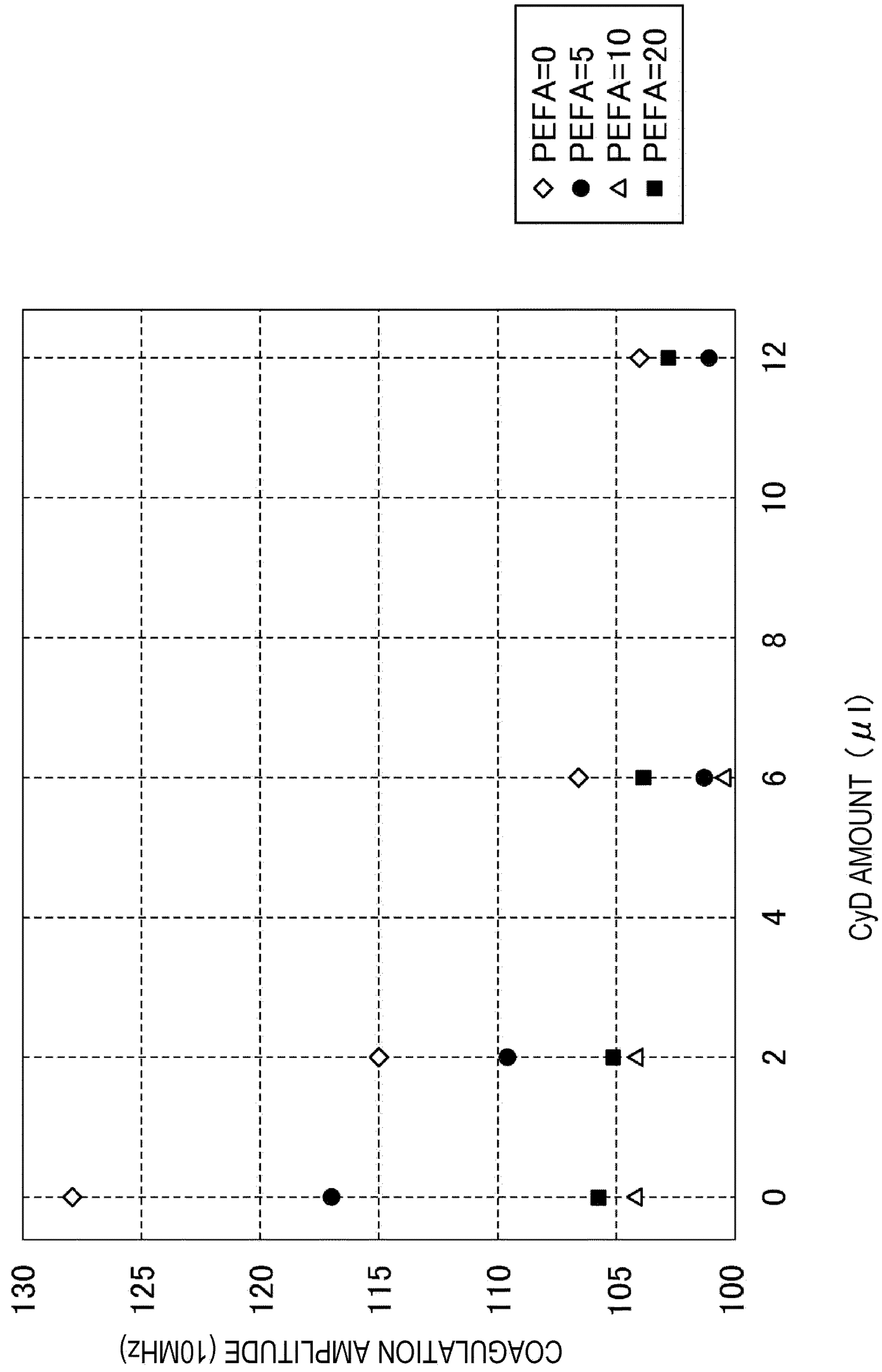
PEFA=0
PEFA=5
PEFA=10
PEFA=20
COAGULATION AMPLITUDE (10MHz)
130
125
120
115
110
105
100
0
2
4
6
8
10
12
CyD AMOUNT（μl）

FIG. 25

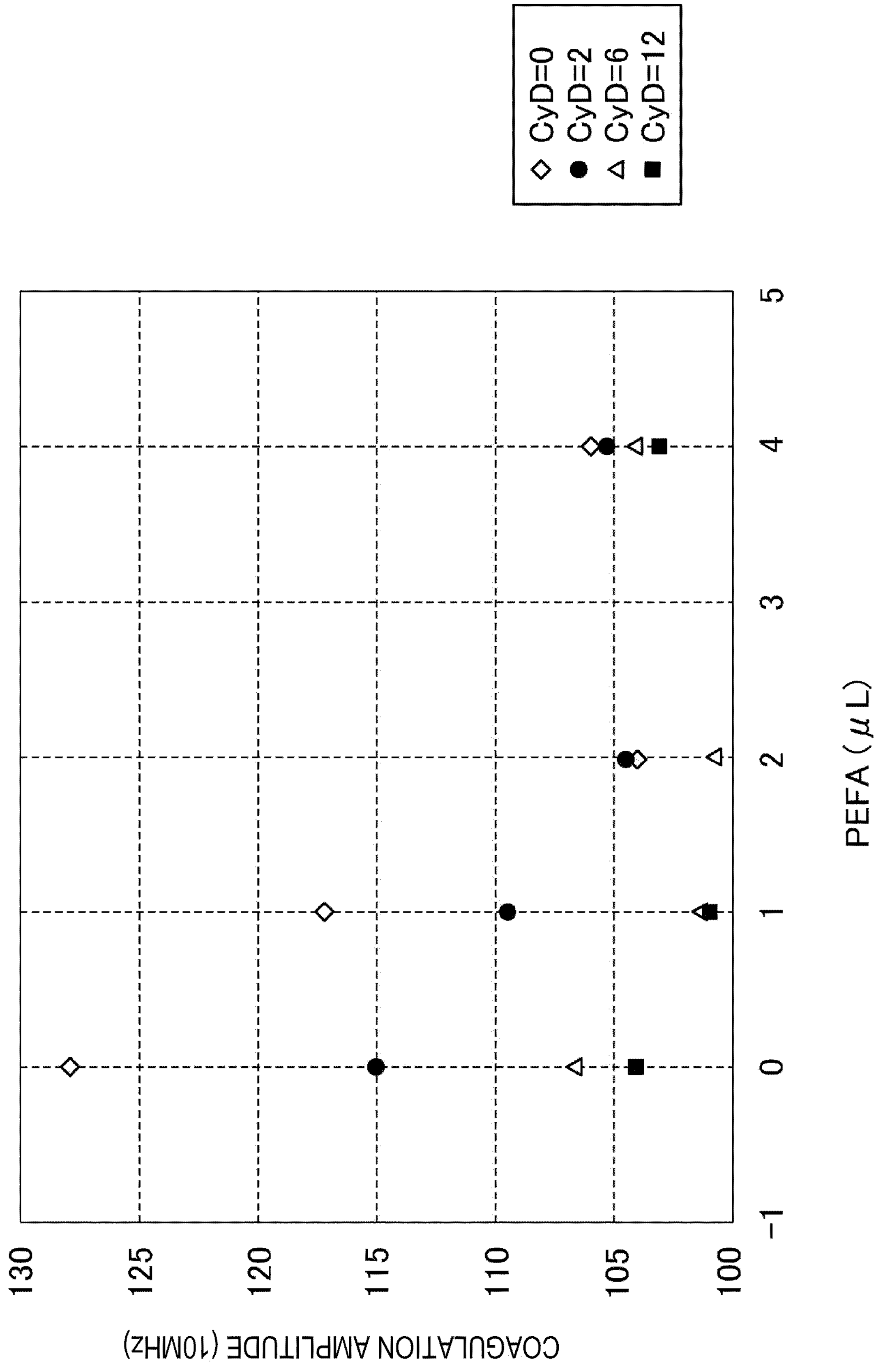
CyD=0
CyD=2
CyD=6
CyD=12
COAGULATION AMPLITUDE (10MHz)
130
125
120
115
110
105
100
PEFA (μL)
−1
0
1
2
3
4
5

# FIG. 26

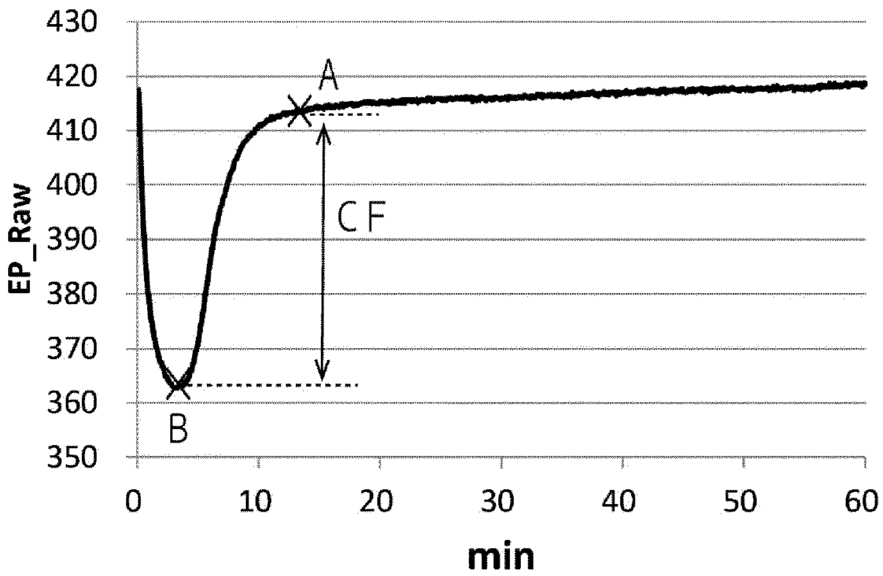
430
420
410
400
390
380
370
360
350
EP_Raw
A
CF
B
0
10
20
30
40
50
60
min

# FIG. 27

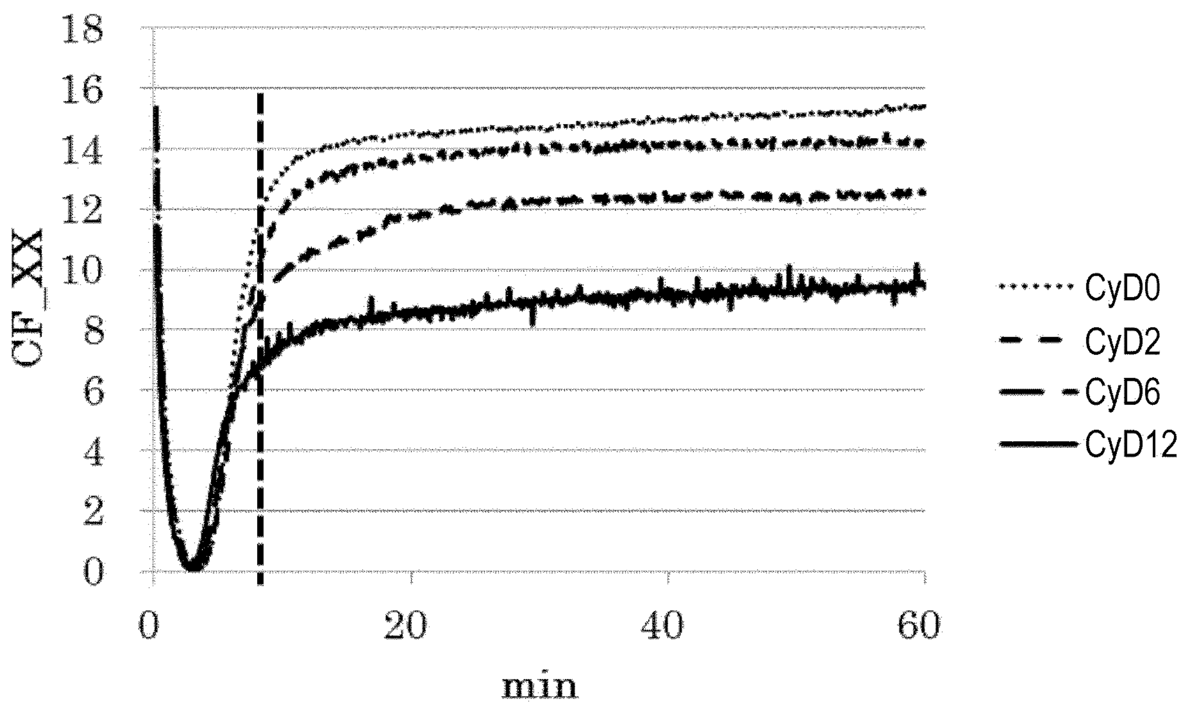
18
16
14
12
10
8
6
4
2
0
CF_XX
0
20
40
60
min
CyD0
CyD2
CyD6
CyD12

FIG. 28

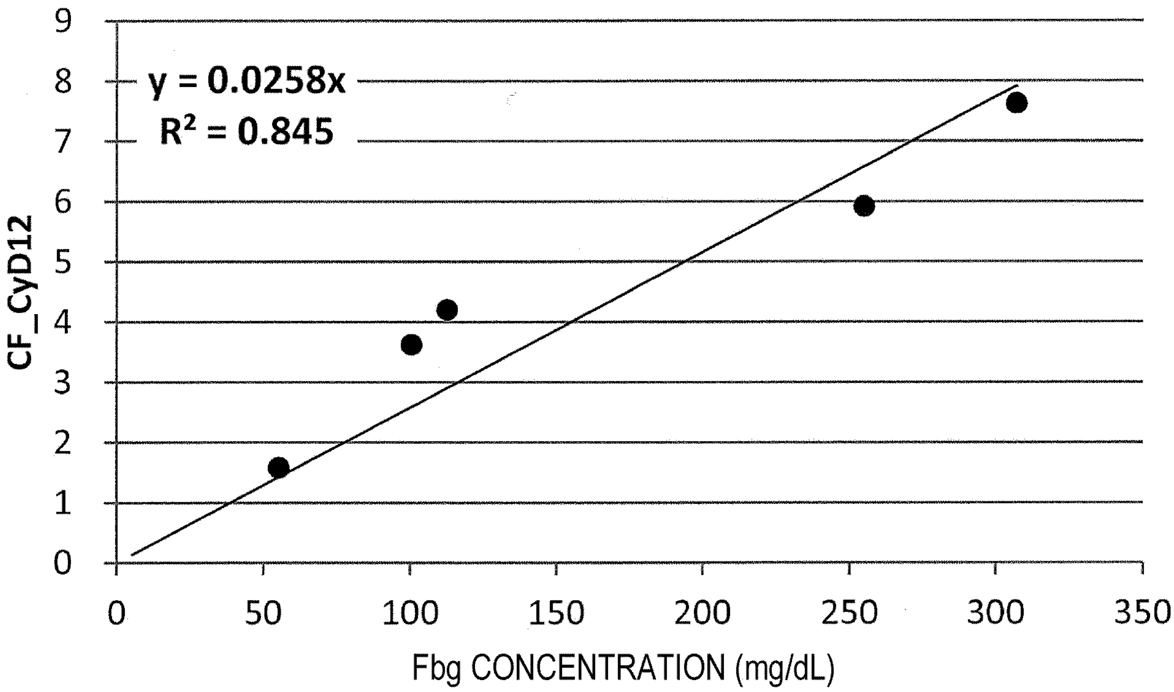
y = 0.0258x
$R^2$ = 0.845
CF_CyD12
Fbg CONCENTRATION (mg/dL)
0
1
2
3
4
5
6
7
8
9
0
50
100
150
200
250
300
350

FIG. 29

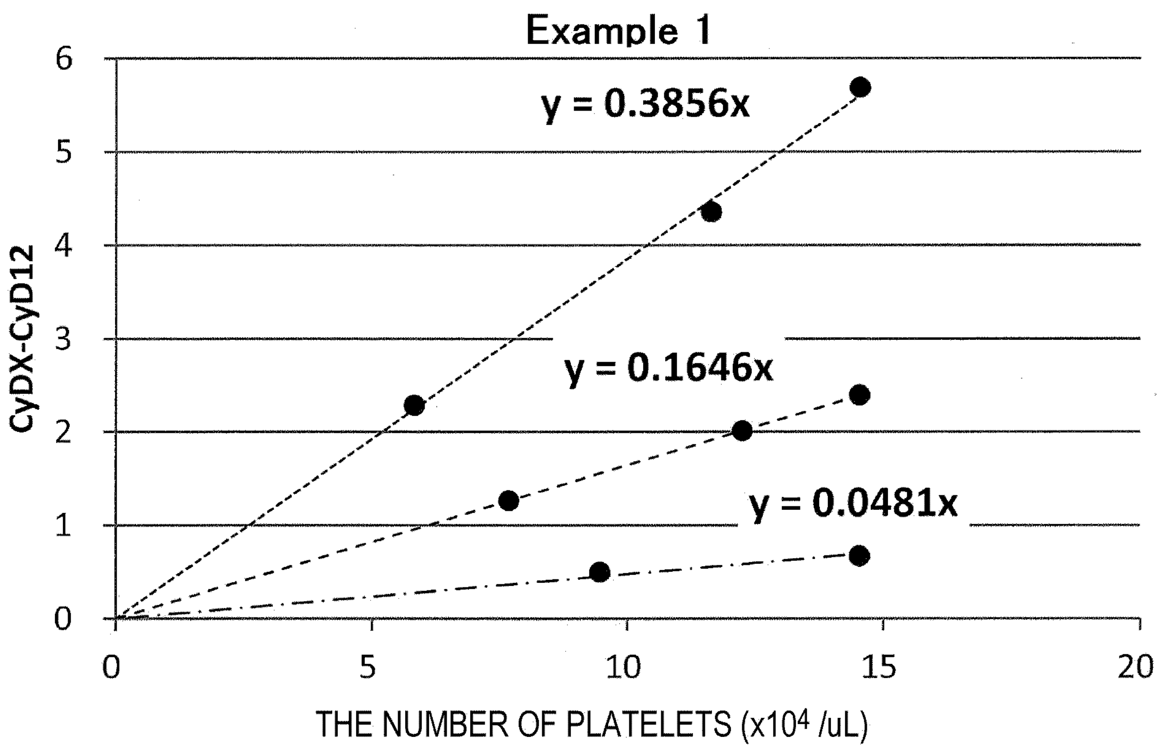
Example 1
y = 0.3856x
y = 0.1646x
y = 0.0481x
CyDX-CyD12
THE NUMBER OF PLATELETS (x10⁴ /uL)
0
1
2
3
4
5
6
0
5
10
15
20

FIG. 30

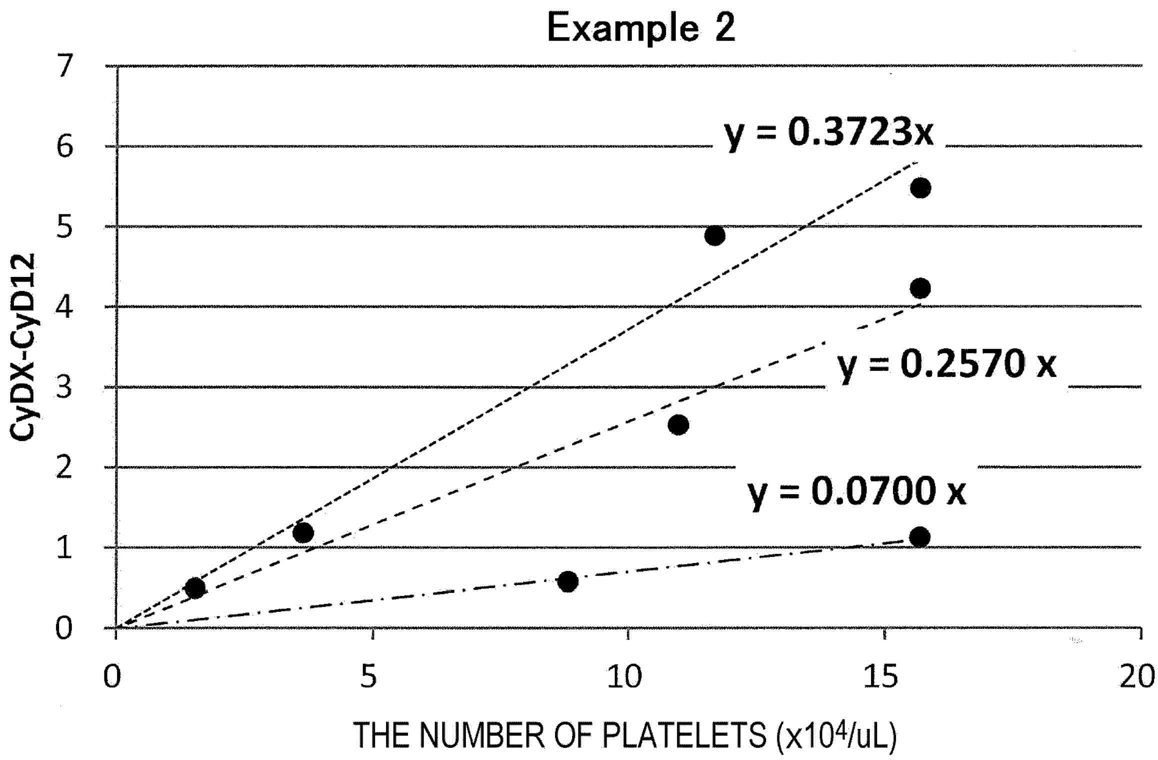
Example 2
y = 0.3723x
y = 0.2570 x
y = 0.0700 x
CyDX-CyD12
7
6
5
4
3
2
1
0
0
5
10
15
20
THE NUMBER OF PLATELETS (x10⁴/uL)

FIG. 31

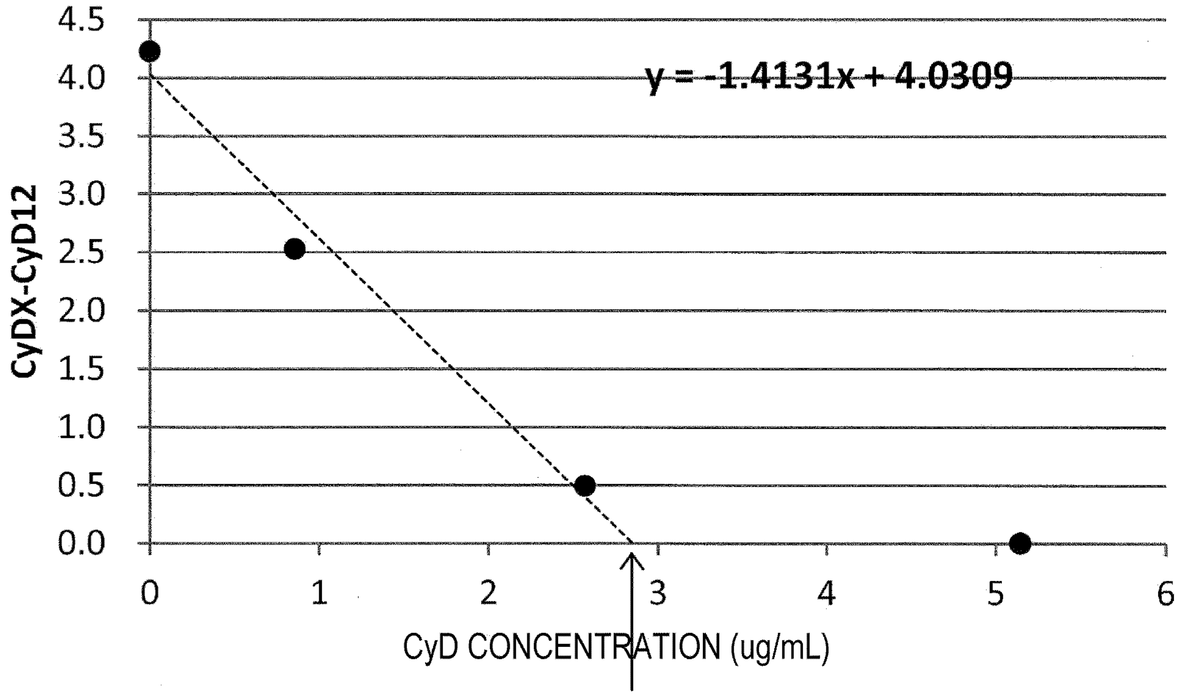
y = -1.4131x + 4.0309
CyDX-CyD12
4.5
4.0
3.5
3.0
2.5
2.0
1.5
1.0
0.5
0.0
0
1
2
3
4
5
6
CyD CONCENTRATION (ug/mL)

*FIG. 32*

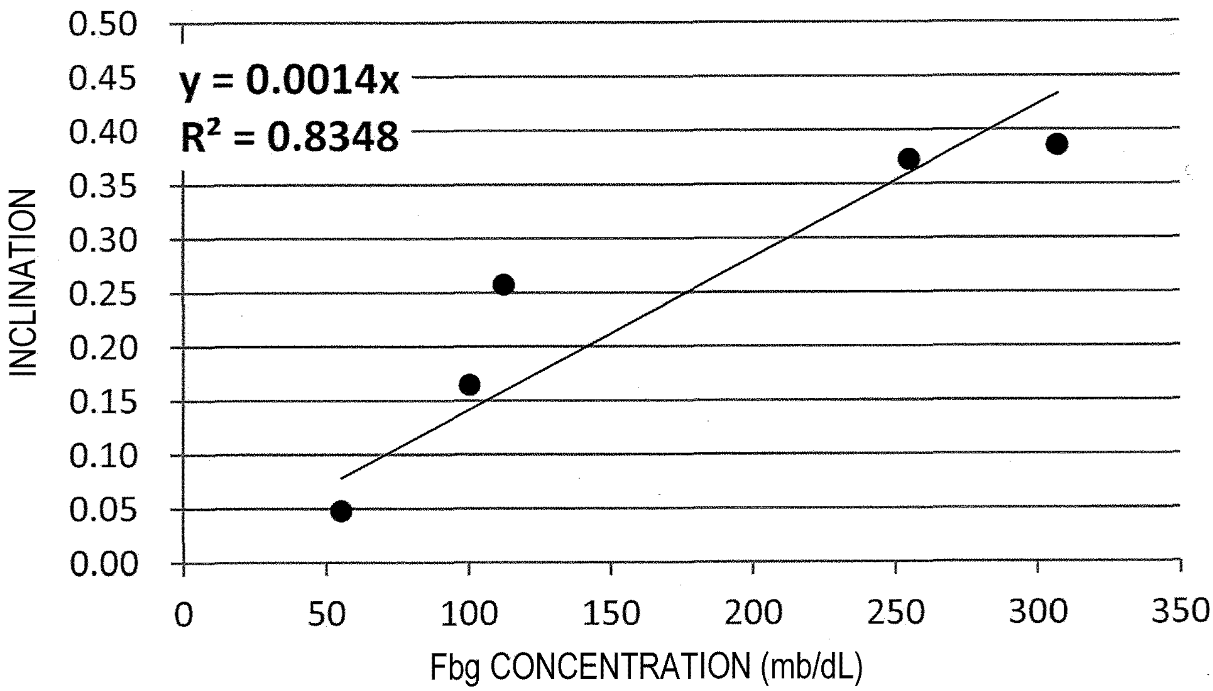
0.50
0.45
0.40
0.35
0.30
0.25
0.20
0.15
0.10
0.05
0.00
INCLINATION
y = 0.0014x
R² = 0.8348
0
50
100
150
200
250
300
350
Fbg CONCENTRATION (mb/dL)

*FIG. 33*

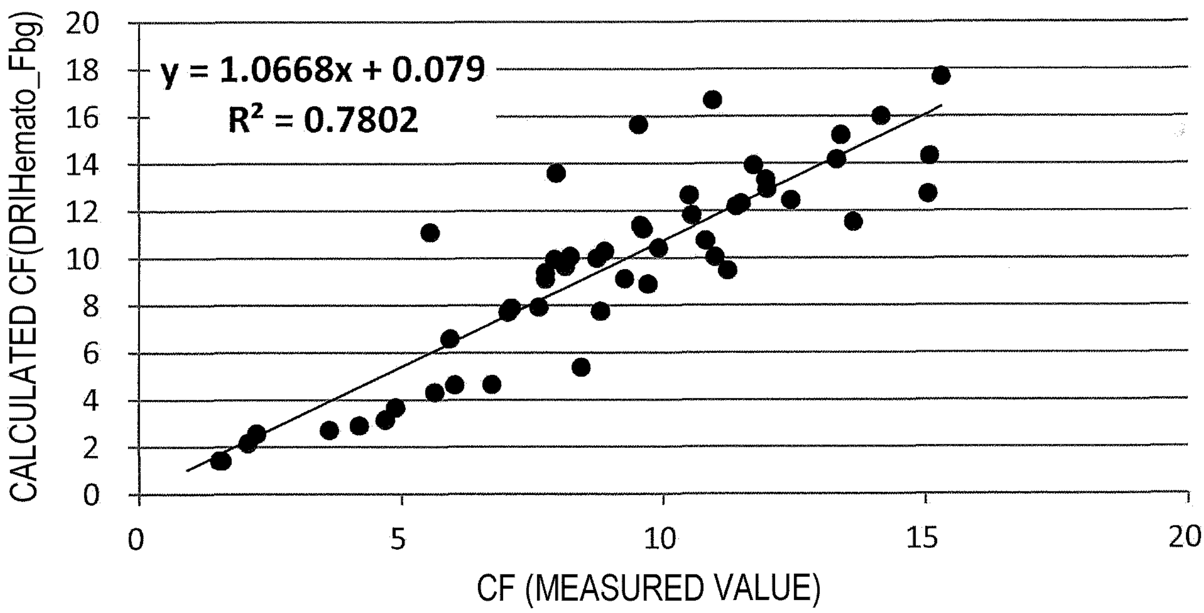
20
18
16
14
12
10
8
6
4
2
0
CALCULATED CF(DRIHemato_Fbg)
y = 1.0668x + 0.079
R² = 0.7802
0
5
10
15
20
CF (MEASURED VALUE)

FIG. 34

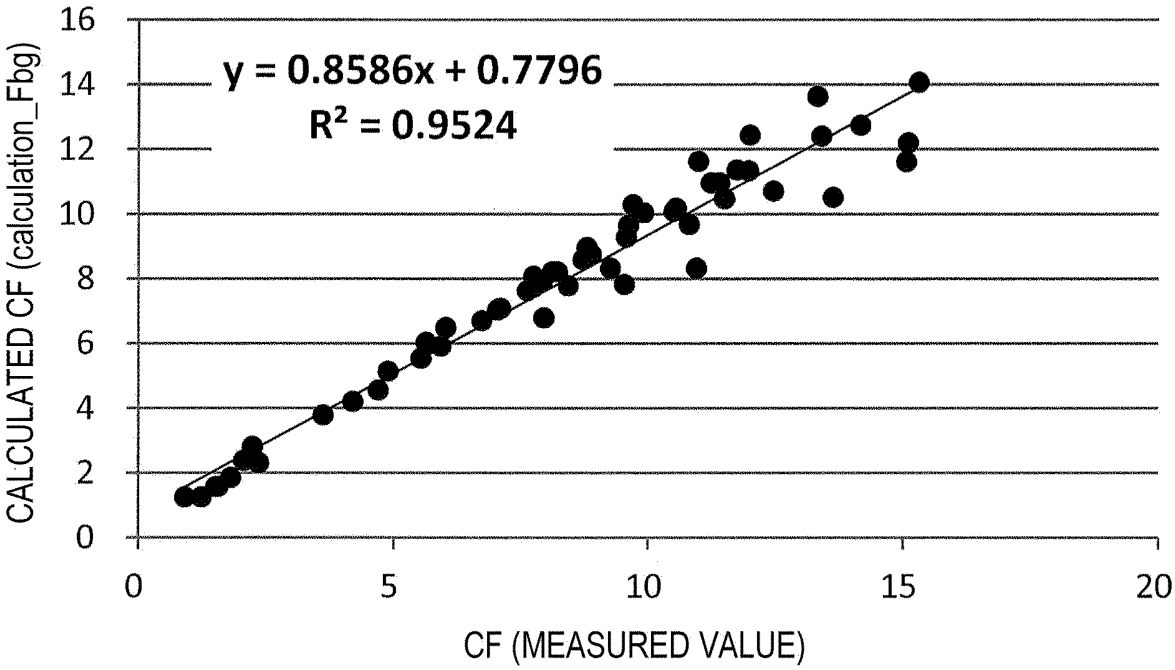
y = 0.8586x + 0.7796
$R^2$ = 0.9524
CALCULATED CF (calculation_Fbg)
CF (MEASURED VALUE)
0
2
4
6
8
10
12
14
16
0
5
10
15
20

FIG. 35

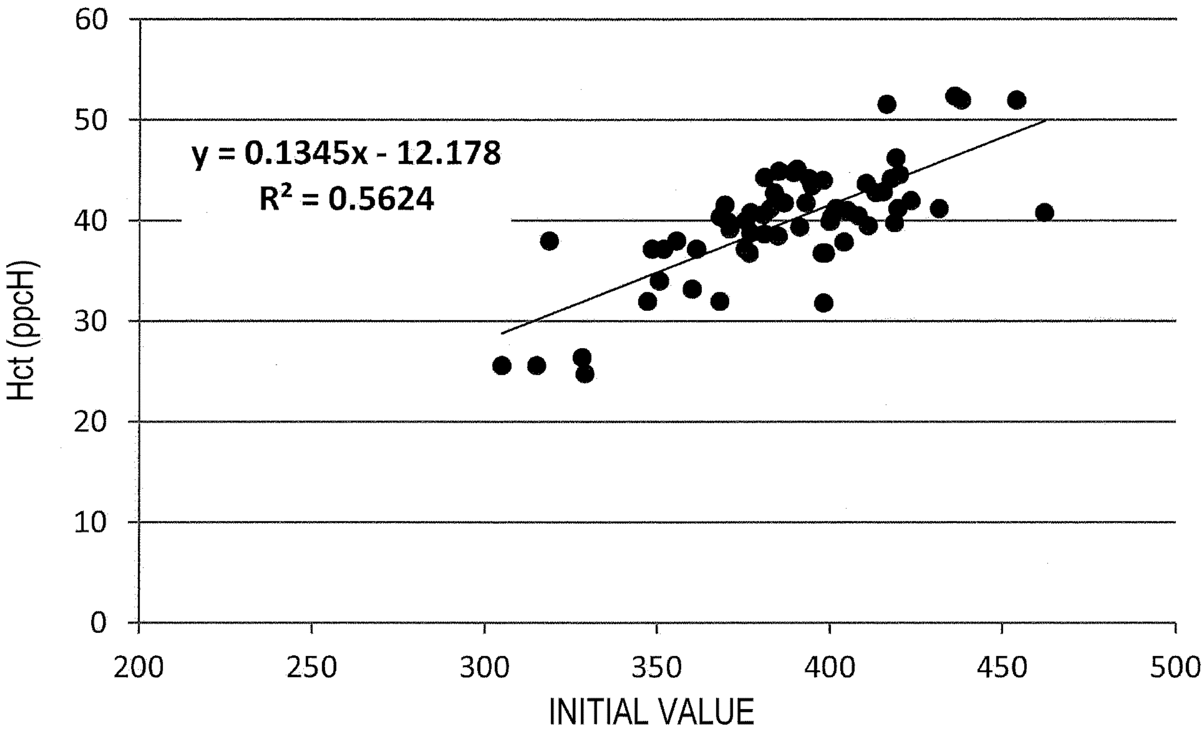
y = 0.1345x - 12.178
$R^2$ = 0.5624
Hct (ppcH)
INITIAL VALUE
0
10
20
30
40
50
60
200
250
300
350
400
450
500

FIG. 36

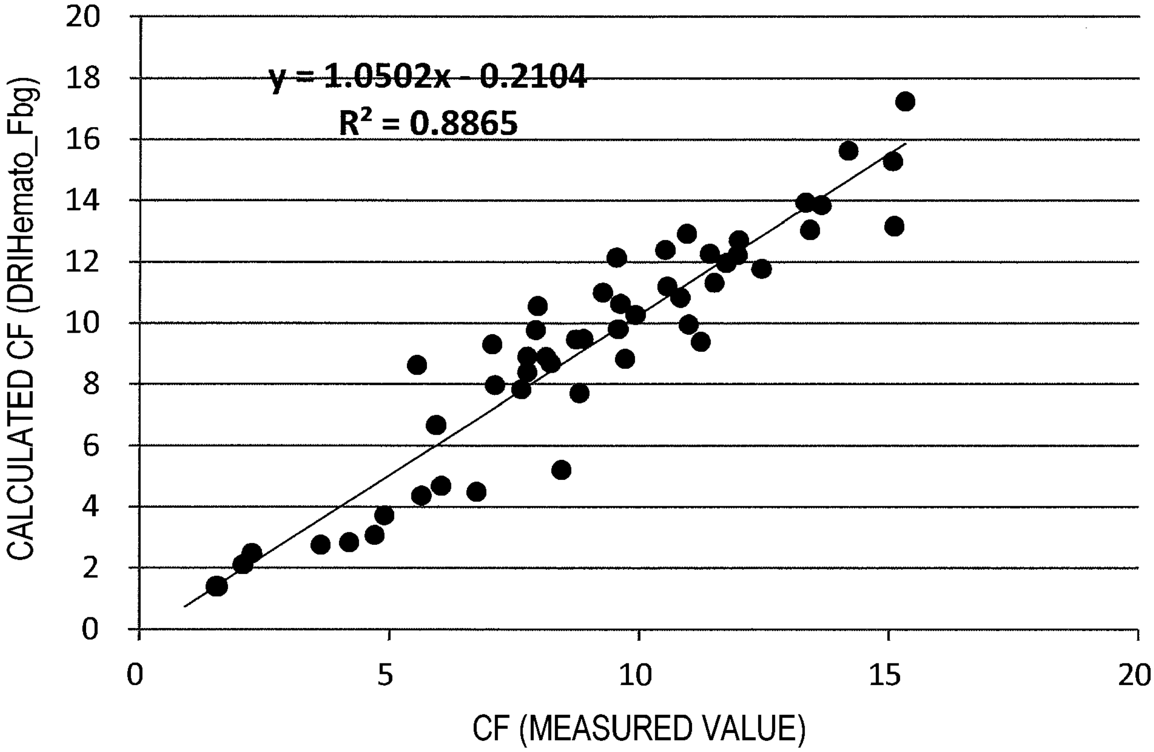
y = 1.0502x - 0.2104
R² = 0.8865
CALCULATED CF (DRIHemato_Fbg)
CF (MEASURED VALUE)
0
2
4
6
8
10
12
14
16
18
20
0
5
10
15
20

FIG. 37

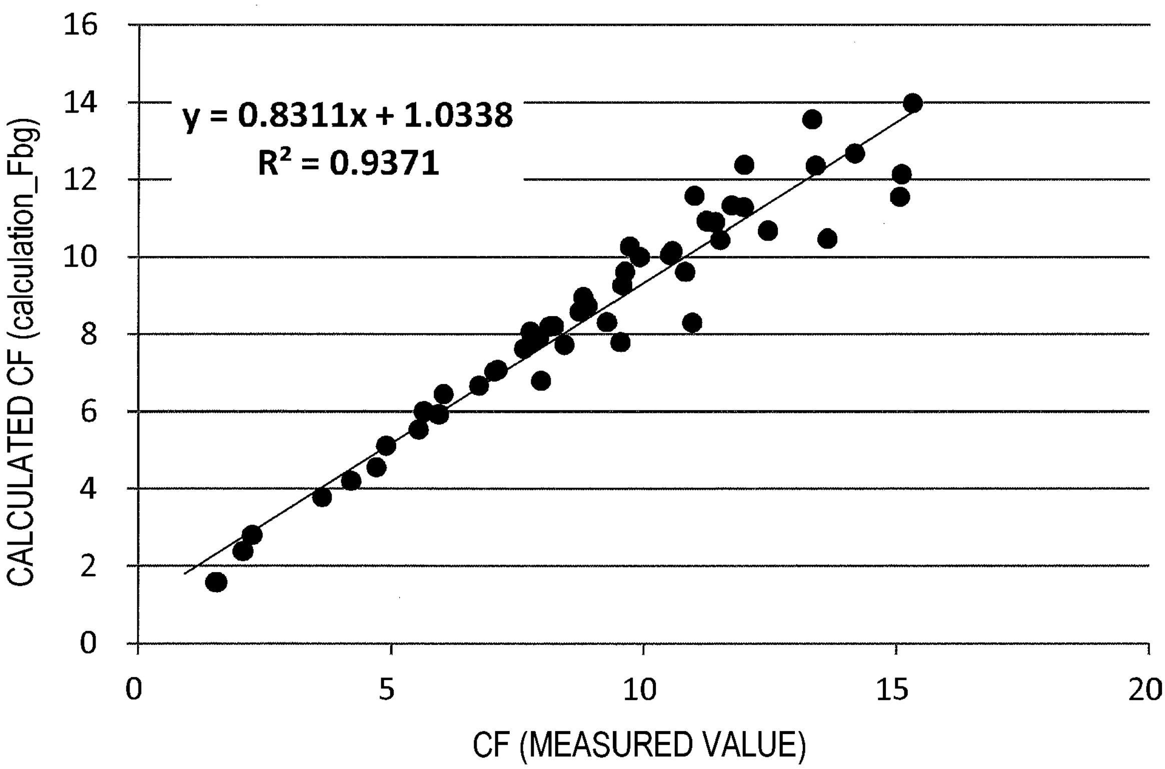
y = 0.8311x + 1.0338
R² = 0.9371
CALCULATED CF (calculation_Fbg)
CF (MEASURED VALUE)
0
2
4
6
8
10
12
14
16
0
5
10
15
20

FIG. 38

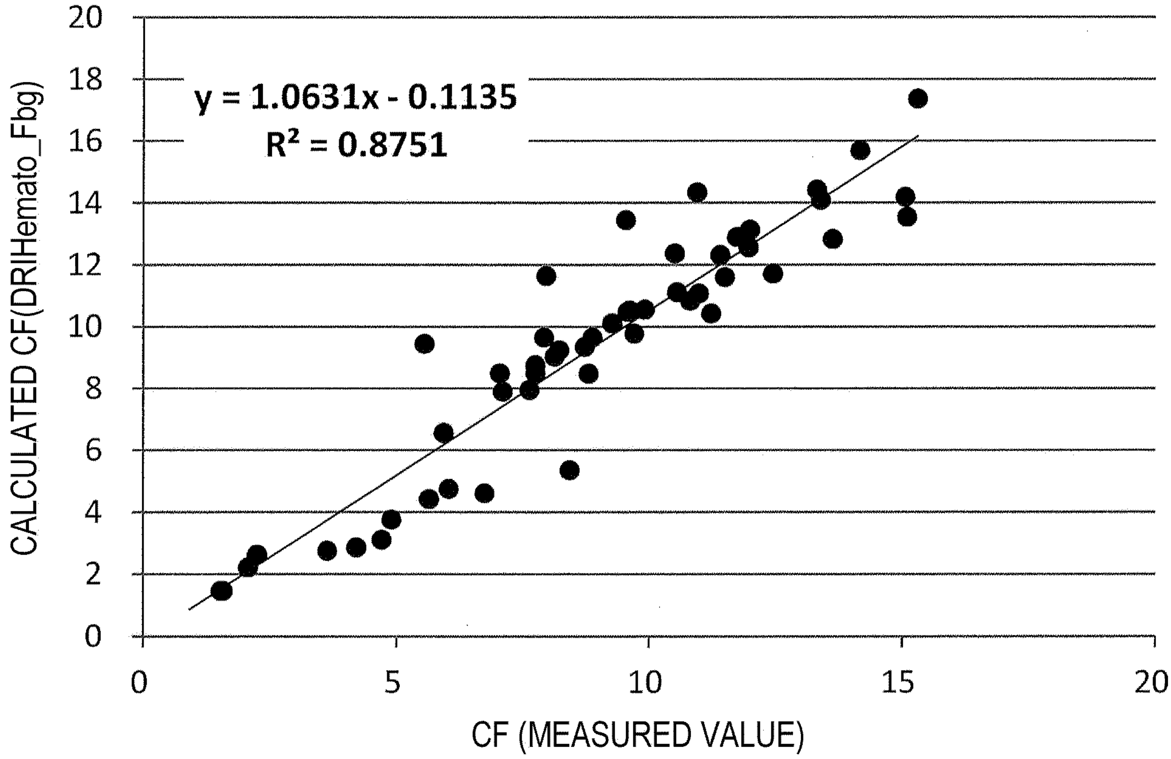
y = 1.0631x - 0.1135
$R^2$ = 0.8751
CALCULATED CF(DRIHemato_Fbg)
CF (MEASURED VALUE)
0
2
4
6
8
10
12
14
16
18
20
0
5
10
15
20

FIG. 39

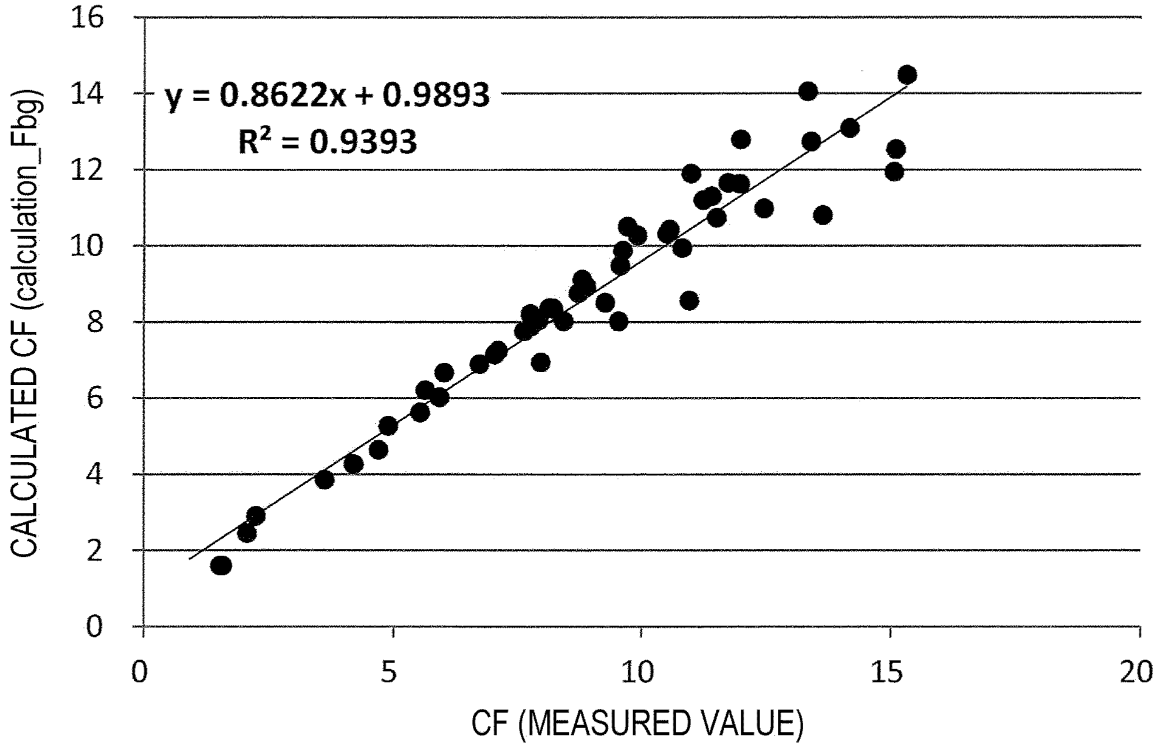
y = 0.8622x + 0.9893
$R^2$ = 0.9393
CALCULATED CF (calculation_Fbg)
CF (MEASURED VALUE)
0
2
4
6
8
10
12
14
16
0
5
10
15
20

## INTERNATIONAL SEARCH REPORT

International application No.
PCT/JP2015/057647

A. CLASSIFICATION OF SUBJECT MATTER
*G01N27/22*(2006.01)i, *G01N27/02*(2006.01)i, *G01N27/06*(2006.01)i, *G01N33/49*(2006.01)i, *G01N33/86*(2006.01)i

According to International Patent Classification (IPC) or to both national classification and IPC

B. FIELDS SEARCHED

Minimum documentation searched (classification system followed by classification symbols)
G01N27/00-27/24, G01N33/49, G01N33/86

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2010/079845 A1 (Sony Corp.),<br>15 July 2010 (15.07.2010),<br>entire text; all drawings<br>& US 2012/0035450 A1 & EP 2375244 A1<br>& CN 102308203 A | 1-16<br>2 |
| Y | JP 2007-64916 A (Tosoh Corp.),<br>15 March 2007 (15.03.2007),<br>paragraphs [0020] to [0021]<br>(Family: none) | 2 |
| A | JP 2012-194087 A (Sony Corp.),<br>11 October 2012 (11.10.2012),<br>entire text; all drawings<br>& US 2012/0238026 A1 & US 2014/0186217 A<br>& EP 2500726 A1 & CN 102680523 A | 1-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 May 2015 (21.05.15) | 02 June 2015 (02.06.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No. PCT/JP2015/057647

C (Continuation). DOCUMENTS CONSIDERED TO BE RELEVANT

| Category* | Citation of document, with indication, where appropriate, of the relevant passages | Relevant to claim No. |
|---|---|---|
| A | JP 2013-221782 A (Sony Corp.), 28 October 2013 (28.10.2013), entire text; all drawings & WO 2013/153735 A & CN 104204787 A | 1-16 |
| A | JP 7-43388 B2 (Hitachi Chemical Co., Ltd.), 15 May 1995 (15.05.1995), entire text; all drawings (Family: none) | 1-16 |
| A | JP 4921359 B2 (Kissei Pharmaceutical Co., Ltd.), 25 April 2012 (25.04.2012), paragraphs [0002] to [0003] (Family: none) | 1-16 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010513905 A
- JP 2010518371 A
- WO 2010079845 A